(19) Europäisches Patentamt
European Patent Office
Office européen des brevets

(11) **EP 3 210 529 B1**

(12) **EUROPEAN PATENT SPECIFICATION**

(45) Date of publication and mention of the grant of the patent:
**06.01.2021 Bulletin 2021/01**

(21) Application number: **15852390.2**

(22) Date of filing: **01.07.2015**

(51) Int Cl.:
**A61B 5/022** *(2006.01)*

(86) International application number:
**PCT/JP2015/068999**

(87) International publication number:
**WO 2016/063577 (28.04.2016 Gazette 2016/17)**

(54) **BLOOD PRESSURE MEASUREMENT METHOD, BLOOD PRESSURE MEASUREMENT DEVICE, BLOOD PRESSURE MEASUREMENT PROGRAM AND COMPUTER-READABLE STORAGE MEDIUM**

BLUTDRUCKMESSUNGSVERFAHREN, BLUTDRUCKMESSUNGSVORRICHTUNG, BLUTDRUCKMESSUNGSPROGRAMM UND COMPUTERLESBARES SPEICHERMEDIUM

PROCÉDÉ DE MESURE DE PRESSION SANGUINE, DISPOSITIF DE MESURE DE PRESSION SANGUINE, PROGRAMME DE MESURE DE PRESSION SANGUINE ET MOYEN DE STOCKAGE LISIBLE PAR ORDINATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.10.2014 JP 2014213567**

(43) Date of publication of application:
**30.08.2017 Bulletin 2017/35**

(73) Proprietor: **Hamamatsu Photonics K.K.**
**Hamamatsu-shi, Shizuoka 435-8558 (JP)**

(72) Inventors:
• **NAKAZAWA, Tomoya**
**Hamamatsu-shi**
**Shizuoka 435-8558 (JP)**
• **SEKINE, Rui**
**Hamamatsu-shi**
**Shizuoka 435-8558 (JP)**

(74) Representative: **Boult Wade Tennant LLP**
**Salisbury Square House**
**8 Salisbury Square**
**London EC4Y 8AP (GB)**

(56) References cited:
WO-A1-01/70106          JP-A- H08 229 011
JP-A- 2003 000 555       US-A- 5 776 071
US-A1- 2005 119 578      US-A1- 2012 277 602

• MOHAMED ELGENDI: "On the Analysis of Fingertip Photoplethysmogram Signals", CURRENT CARDIOLOGY REVIEWS, vol. 8, no. 1, 1 June 2012 (2012-06-01), pages 14-25, XP55206723, ISSN: 1573-403X, DOI: 10.2174/157340312801215782

EP 3 210 529 B1

**Description**

**Technical Field**

An aspect of the present invention relates to a blood pressure measurement method, a blood pressure measurement device, a blood pressure measurement program, and a computer-readable storage medium.

**Background Art**

Conventionally, as a non-invasive blood pressure measurement method that reduces the burden on a test subject, a method of obtaining a blood pressure value by analyzing a pulse wave is known. For example, in a blood pressure measurement method described in patent application JP 2006-6897, an acceleration pulse wave is obtained by secondary differentiation of a volume pulse wave and a value of a diastolic blood pressure is estimated on the basis of a time interval between two feature points in the acceleration pulse wave. Also, for example, in a blood pressure measurement method described in patent application JP 11-155826, various feature quantities associated with a blood pressure are calculated from waveforms of a volume pulse wave, a velocity pulse wave obtained by first differentiation of the volume pulse wave, and an acceleration pulse wave obtained by secondary differentiation of the volume pulse wave and a value of a diastolic blood pressure or a systolic blood pressure is calculated on the basis of various feature quantities that are calculated.

As a non-invasive blood pressure measurement method, a method of obtaining a series of time-varying blood pressure values as well as a blood pressure value at a predetermined time is also known. For example, in the blood pressure measurement method described in patent application JP 2001-187032 , infrared light is transmitted into an inside of the body, a frequency of a reflected wave from the inside of the body is acquired, and a blood pressure is measured by performing calibration based on the frequency. Specifically, this calibration is performed by associating a maximum frequency and a minimum frequency of the frequency of the reflected wave from the inside of the body with a maximum blood pressure (systolic blood pressure) and a minimum blood pressure (diastolic blood pressure) measured in advance by a cuff compression method or the like and approximating blood pressure values from a linear function related to a frequency.

Patent application US 2012/0277602 discloses another system and method for monitoring a subject's blood pressure, wherein the dicrotic notch, as present in the pressure waveform detected by a pressure sensor, is used as a landmark for aligning a pulse waveform detected by a pulse sensor. The aligned waveforms are then used to continuously monitor the subject's diastolic and systolic blood pressure.

**Summary of Invention**

**Technical Problem**

In the blood pressure measuring methods described in the above-described JP 2006-6897 and JP 11-155826, only a blood pressure value at a predetermined time such as a diastolic blood pressure or a systolic blood pressure can be measured, and a series of time-varying blood pressure values cannot be obtained.

In the blood pressure measurement method described in the above-described JP 2001-187032 , a series of time-varying blood pressure values according to calibration can be obtained, but it is necessary to perform complex calibration in each motional state of a test subject because differences occur in frequency of a reflected wave from the inside of the body according to the motional state of the test subject (e.g., at the time of rest, after exercise, or the like).

An aspect of the present invention is to provide a blood pressure measurement method and a blood pressure measurement device capable of accurately and easily obtaining a series of time-varying blood pressure values.

**Solution to Problem**

To solve the above-described problem, the present inventors newly found the following facts from results of intensive research.

A time waveform based on a volume pulse wave can be acquired as a waveform similar to a blood pressure waveform indicating a change in a blood pressure with respect to time and a DN point corresponding to a dicrotic notch point in the blood pressure waveform appears in the time waveform based on the volume pulse wave. The dicrotic notch point in the blood pressure waveform is a point of change in the blood pressure caused by the closure of the arterial valve of the heart due to a decrease in a blood flow rate and the blood pressure value at this point of change is substantially constant irrespective of the motional state of the test subject. Consequently, the present inventors found that a waveform corresponding to the blood pressure waveform can be acquired by correcting the time waveform so that the time waveform value of the DN point in the time waveform becomes a reference value by using the blood pressure value at the dicrotic notch point in the blood pressure waveform as the reference value.

An aspect of the present invention based on the above-described fact is a blood pressure measurement method of acquiring a change in a blood pressure with respect to time, as defined in the appended claims 1 to 6.

In the blood pressure measurement method according to the above aspect, in the time waveform, a DN point corresponding to a dicrotic notch point in the blood pressure waveform is detected and the time waveform is corrected so that the time waveform value of the DN point becomes a predetermined blood pressure value. Consequently, for example, using the blood pressure value of the dicrotic notch point in the pre-measured blood pressure waveform as a reference value, it is possible to obtain a waveform corresponding to the blood pressure waveform by correcting the time waveform so that the time waveform value of the DN point in the time waveform becomes the reference value. Thereby, a series of time-varying blood pressure values can be accurately and easily obtained.

Another aspect of the present invention is a blood pressure measurement device for acquiring a change in a blood pressure with respect to time, as defined in the appended claims 7 to 13.

Also, another aspect of the present invention is a blood pressure measurement program for causing a computer to execute blood pressure measurement for acquiring a change in a blood pressure with respect to time, as defined in the appended claim 14. Also, another aspect of the present invention is a computer-readable storage medium as defined in the appended claim 15.

The above-described blood pressure measurement device, blood pressure measurement program, and computer-readable storage medium have operations and effects similar to those of the blood pressure measurement method.

## Advantageous Effects of Invention

According to an aspect of the present invention, there are provided a blood pressure measurement method, a blood pressure measurement device, a blood pressure measurement program, and a computer-readable storage medium, which can accurately and easily obtain a series of time-varying blood pressure values.

## Brief Description of Drawings

FIG. 1 is a schematic configuration diagram illustrating a blood pressure measurement system including a computer which is a blood pressure measurement device according to a first example.
FIG. 2 is a functional block diagram of a computer in FIG. 1.
FIG. 3 is a diagram illustrating a method in which a pulse wave acquisition unit acquires a pulse wave waveform.
FIG. 4 is a diagram illustrating a correspondence relationship between a blood pressure waveform and a pulse wave waveform.
FIG. 5 is a conceptual diagram in which an aortic valve is physically modeled.
FIG. 6 is a diagram illustrating a first correction process in a method in which a waveform correction unit corrects a pulse wave waveform.
FIG. 7 is a diagram illustrating a second correction process in a method in which the waveform correction unit corrects a pulse wave waveform.
FIG. 8 is a hardware configuration diagram of a computer in FIG. 1.
FIG. 9 is a flowchart illustrating a blood pressure measurement method according to a first example.
FIG. 10 is a flowchart illustrating a detailed procedure in a waveform correction step illustrated in FIG. 9.
FIG. 11 is a schematic configuration diagram illustrating a blood pressure measurement device according to a second example.
FIG. 12 is a schematic configuration diagram illustrating a blood pressure measurement system according to a modified example.
FIG. 13 is a graph illustrating a blood pressure waveform measured by an invasive catheter sphygmomanometer.
FIG. 14 is a graph illustrating a power spectrum of a blood pressure waveform illustrated in FIG. 13.
FIG. 15 is a graph illustrating a change in blood pressure of a cynomolgus monkey due to an anesthetic agent.
FIG. 16 is a graph illustrating a correlation between a ratio between highest blood pressure and lowest blood pressure and a ratio based on a spectral intensity of the blood pressure waveform.
FIG. 17 is a graph illustrating a spread of a power spectrum of a blood pressure waveform due to living body fluctuation.
FIG. 18 is a diagram illustrating an effective width of the spectral intensity of the power spectrum illustrated in FIG. 17. **Description of** Examples

Hereinafter, examples will be described in detail with reference to the accompanying drawings. The same elements or elements having the same function are denoted by the same reference signs in the description, and redundant description thereof will be omitted.

(First Embodiment)

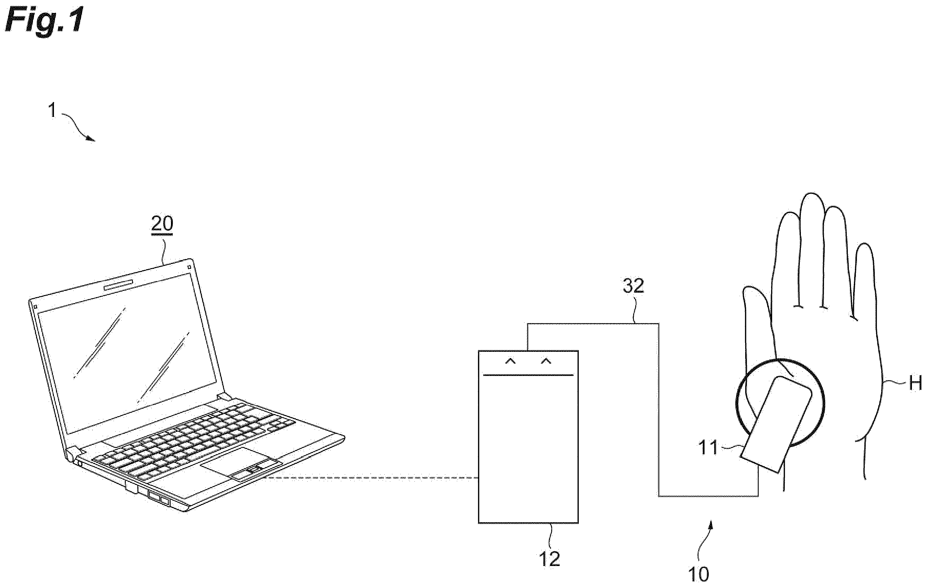

FIG. 1 is a schematic configuration diagram illustrating a blood pressure measurement system including a blood pressure measurement device according to a first example. As illustrated in FIG. 1, the blood pressure measurement system 1 includes a pulse wave measurement device 10 and a computer 20 (blood pressure measurement device).

The pulse wave measurement device 10 measures a volume pulse wave in a living body serving as a test subject (a subject whose blood pressure is measured) by using, for example, near infrared spectroscopy called so-called near infra-red spectroscopy (NIRS). A volume pulse wave shows a waveform in which a change in a blood flow rate occurring at a predetermined position in a living body with respect to time is measured on the surface of the living body. The pulse wave measurement device 10 includes a probe 11 and a measurement unit 12. The probe 11 is attached to a surface of a living body H serving as a test subject (a palm in the present example) . The probe 11 has a light source and a photodetector and radiates near infrared light from the light source from the surface of the living body H to the inside thereof and detects reflected light from the inside of the living body H with the photodetector. Thereby, an absorbance when light passes through the inside of the living body H is obtained. Because this absorbance varies with a blood flow rate at a position where the probe 11 is attached to the living body H, the change in this absorbance with respect to time corresponds to the volume pulse wave. Examples of components that absorb light in a blood flow include red blood cells, hemoglobin contained in red blood cells, moisture, and the like. The probe 11 outputs a signal indicating the detected absorbance to the measurement unit 12.

The measurement unit 12 is connected to the probe 11 through a cable 32 and controls the probe 11. The measurement unit 12 receives a signal indicating the absorbance detected by the probe 11 and measures the absorbance with respect to time. Thereby, the measurement unit 12 measures a volume pulse wave. The measurement unit 12 transmits information indicating the measured volume pulse wave to the computer 20 immediately by wireless communication or at predetermined intervals. The measurement unit 12 may transmit the information to the computer 20 by wire communication via a cable or the like.

The computer 20 receives information indicating the volume pulse wave transmitted from the measurement unit 12 and performs a waveform correction process to be described below with respect to the volume pulse wave on the basis of the received information. As a result, the computer 20 acquires a waveform corresponding to a blood pressure waveform indicating a change in a blood pressure with respect to time. Hereinafter, each function of the computer 20 will be described in detail.

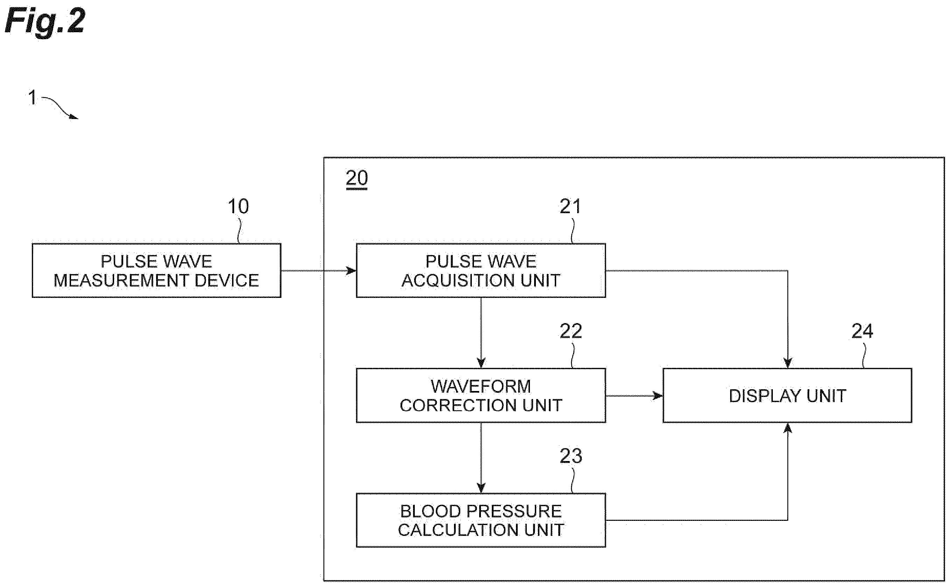

FIG. 2 is a functional block diagram of the computer 20 in FIG. 1. As illustrated in FIG. 2, the computer 20 includes a pulse wave acquisition unit 21, a waveform correction unit 22, a blood pressure calculation unit 23, and a display unit 24.

The pulse wave acquisition unit 21 acquires a time waveform based on the volume pulse wave on the basis of the information indicating the volume pulse wave transmitted from the pulse wave measurement device 10. The time waveform based on the volume pulse wave is a volume pulse wave waveform which is a waveform of the volume pulse wave or a waveform obtained by differentiating the volume pulse wave with respect to time. That is, the time waveform may be the waveform itself of the volume pulse wave or may be a waveform obtained by differentiating the volume pulse wave with respect to time. Also, the differentiation of the volume pulse wave with respect to time may be performed a plurality of times. In the following description, a waveform obtained by performing first-order differentiation on the volume pulse wave with respect to time is defined as a time waveform based on the volume pulse wave and is simply referred to as a "pulse wave waveform." A time waveform value (waveform intensity value) at any point in the time waveform is simply referred to as a "pulse wave waveform value."

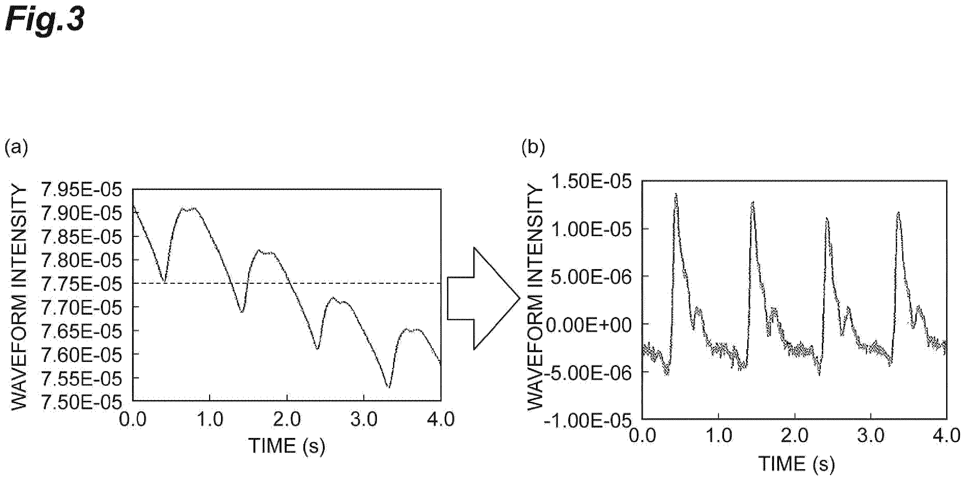

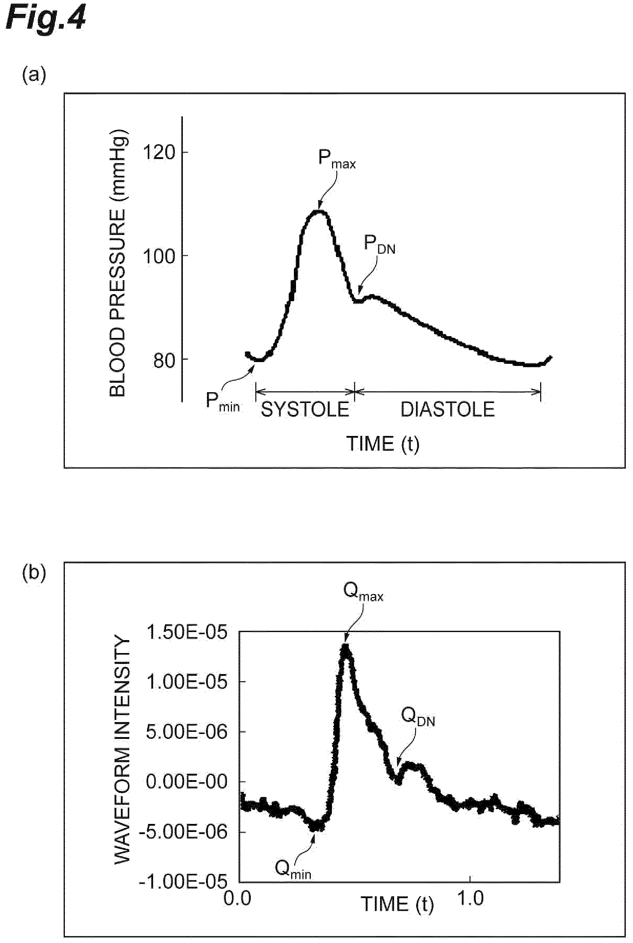

Here, a method of obtaining a pulse wave waveform by performing first-order differentiation on the volume pulse wave with respect to time will be described with reference to FIG. 3. Part (a) of FIG. 3 is a graph illustrating a volume pulse wave measured by the pulse wave measurement device 10, wherein the horizontal axis represents time and the vertical axis represents a waveform intensity of the volume pulse wave. Part (b) of FIG. 3 is a graph illustrating a pulse wave waveform in which the horizontal axis represents time and the vertical axis represents a waveform intensity of the pulse wave waveform. As illustrated in FIG. 3, the volume pulse wave and the pulse wave waveform have a shape that depends on a blood pressure waveform (see part (b) of FIG. 4) showing a change in a blood pressure with respect to time. Particularly, the pulse wave waveform illustrated in part (b) of FIG. 3 has a shape very similar to the blood pressure waveform showing the change in the blood pressure with respect to time. Details of the correspondence relationship between the blood pressure waveform and the pulse wave waveform will be described below in the description of the waveform correction unit 22. The pulse wave acquisition unit 21 outputs the acquired pulse wave waveform to the waveform correction unit 22 and the display unit 24.

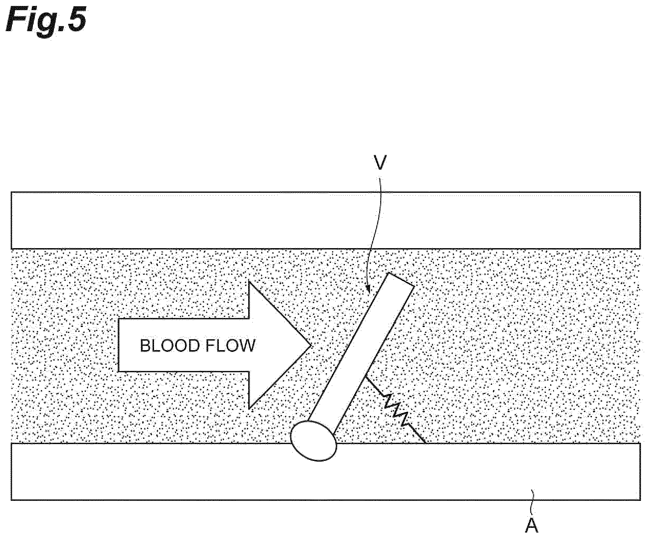

The waveform correction unit 22 corrects the pulse wave waveform output from the pulse wave acquisition unit 21. Specifically, the waveform correction unit 22 corrects the pulse wave waveform using the dicrotic notch point as a reference in the blood pressure waveform on the basis of the correspondence relationship between the blood pressure waveform indicating the change in the blood pressure with respect to time and the pulse wave waveform. The dicrotic notch point in the blood pressure waveform is a point of change of the blood pressure caused by the aortic valve closing according to a decrease in a blood flow rate. Hereinafter, the dicrotic notch point will be simply referred to as a "notch point" and the notch point will be described in detail with reference to FIGS. 4 and 5.

First, the correspondence relationship between the blood pressure waveform and the pulse wave waveform will be described with reference to FIG. 4. Part (a) of FIG. 4 is a graph illustrating a blood pressure waveform in which the horizontal axis represents time and the vertical axis represents blood pressure. Part (b) of FIG. 4 is a graph illustrating the pulse wave waveform in which the horizontal axis represents time and the vertical axis represents a waveform intensity of the pulse wave waveform.

As illustrated in FIG. 4, the blood pressure waveform and the pulse wave waveform have shapes very similar to each other. As illustrated in part (a) of FIG. 4, the blood pressure waveform has diastolic blood pressure $P_{min}$ which is lowest in the diastole, systolic blood pressure $P_{max}$ which is highest in the systole, and a notch point $P_{DN}$ which is a point of change caused by the closure of the aortic valve. In correspondence with this, as illustrated in part (b) of FIG. 4, the pulse wave waveform has a minimum point $Q_{min}$ corresponding to the diastolic blood pressure $P_{min}$, a maximum point $Q_{max}$ corresponding to the systolic blood pressure $P_{max}$, and a DN point $Q_{DN}$ corresponding to the notch point $P_{DN}$. As described above, the shapes of the blood pressure waveform and the pulse wave waveform have a correspondence relationship and the shape of the pulse wave waveform depends on the shape of the blood pressure waveform.

Next, the physical meaning of the notch point $P_{DN}$ in the blood pressure waveform will be described with reference to FIG. 5. FIG. 5 is a conceptual diagram in which an aortic valve is physically modeled. As illustrated in FIG. 5, an aortic valve V is physically modeled as a valve having a rotary spring with a predetermined initial load. The aortic valve V is located in a blood outflow path A from a left ventricle of the heart to the aorta. When the blood flow rate is sufficiently high, the aortic valve V is opened by a blood flow. When the blood flow rate becomes equal to or less than the predetermined initial load, the aortic valve V is closed, thereby generating the notch point $P_{DN}$ in the blood pressure waveform. Consequently, the blood pressure value at the moment when the valve closes depends on only the magnitude of the predetermined initial load. Therefore, the blood pressure value at the notch point $P_{DN}$ can be assumed to be a substantially constant value for each test subject irrespective of the motional state of the test subject.

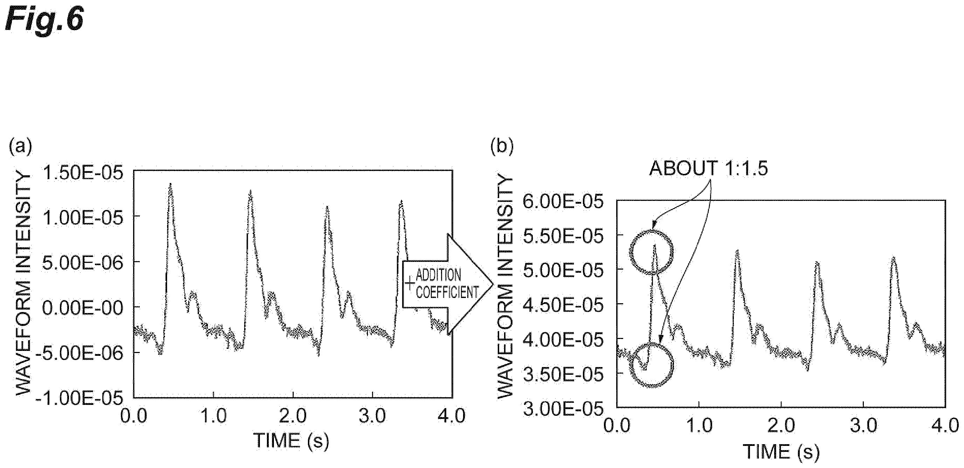

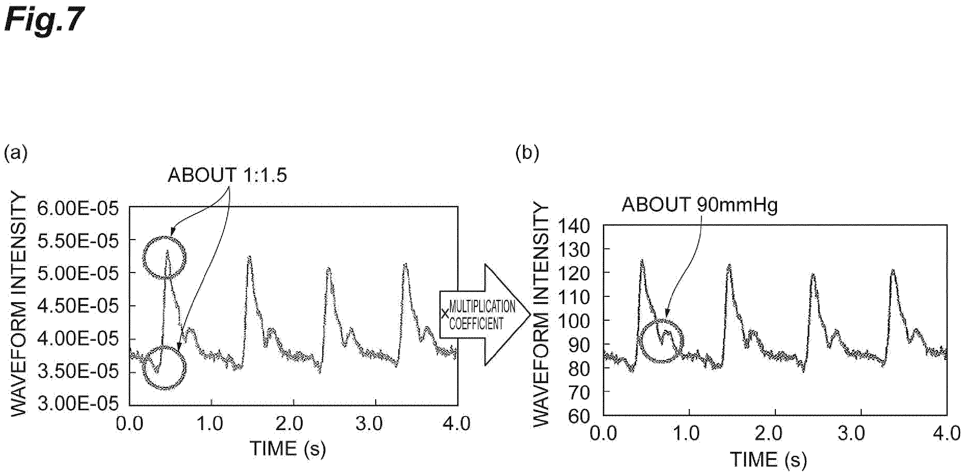

On the basis of the above-described assumption, the waveform correction unit 22 corrects a pulse wave waveform so that a pulse wave waveform value of the DN point $Q_{DN}$ in the pulse wave waveform becomes a reference value by using, for example, the blood pressure value at the notch point $P_{DN}$ measured in advance as the reference value. Specifically, the waveform correction unit 22 performs pulse wave waveform correction including a first correction process and a second correction process as described below. Hereinafter, a method in which the waveform correction unit 22 corrects a pulse wave waveform will be described in detail with reference to FIGS. 6 and 7.

First, the first correction process will be described with reference to FIG. 6. Part (a) of FIG. 6 is a graph illustrating the pulse wave waveform before the first correction process, wherein the horizontal axis represents time and the vertical axis represents a waveform intensity. Part (b) of FIG. 6 is a graph illustrating the pulse wave waveform after the first correction process, wherein the horizontal axis represents time and the vertical axis represents a waveform intensity.

In the first correction process, the waveform correction unit 22 detects the minimum point $Q_{min}$ and the maximum point $Q_{max}$ in the pulse wave waveform acquired by the pulse wave acquisition unit 21. Then, the waveform correction unit 22 calculates the ratio between the detected minimum point $Q_{min}$ and the detected maximum point $Q_{max}$ and adds an addition coefficient to the pulse wave waveform acquired by the pulse wave acquisition unit 21 so that the ratio becomes a predetermined value. Here, the predetermined value is set in advance on the basis of the ratio between the diastolic blood pressure $P_{min}$ and the systolic blood pressure $P_{max}$ in the blood pressure waveform of the test subject calculated or measured in advance. The ratio between the diastolic blood pressure $P_{min}$ and the systolic blood pressure $P_{max}$ in the blood pressure waveform of the test subject may be calculated from, for example, statistical data in advance and may be measured in advance in an indirect method such as a cuff compression method, a direct method such as an invasive method, or the like and may be calculated by performing frequency analysis of the volume pulse wave.

FIG. 6 illustrates an example in which the ratio between the diastolic blood pressure $P_{min}$ and the systolic blood pressure $P_{max}$ in the blood pressure waveform of the test subject is about 1:1.5. In this case, the waveform correction unit 22 adds an addition coefficient to the waveform intensity for each time shown in the pulse wave waveform so that the ratio between the minimum point $Q_{min}$ and the maximum point $Q_{max}$ in the pulse wave waveform is about 1:1.5. Thereby, in the pulse wave waveform after the first correction process, the ratio between the minimum point $Q_{min}$ and the maximum point $Q_{max}$ is about 1:1.5.

Next, the second correction process will be described with reference to FIG. 7. Part (a) of FIG. 7 is a graph illustrating the pulse wave waveform before the second correction process, wherein the horizontal axis represents time and the vertical axis represents a waveform intensity. Part (b) of FIG. 7 is a graph of the pulse wave waveform after the second correction process, wherein the horizontal axis represents time and the vertical axis represents a waveform intensity.

In the second correction process, the waveform correction unit 22 detects the DN point $Q_{DN}$ in the pulse wave waveform after the first correction process. Then, the waveform correction unit 22 multiplies the pulse wave waveform after the first correction process by a multiplication coefficient so that the pulse wave waveform value of the detected DN point $Q_{DN}$ becomes a predetermined blood pressure value. Here, the predetermined blood pressure value is set in advance on the basis of the blood pressure value at the notch point $P_{DN}$ in the blood pressure waveform of the test subject calculated or measured in advance. The blood pressure value at the notch point $P_{DN}$ in the blood pressure waveform of the test subject may be calculated in advance from, for example, statistical data or may be measured in advance by an indirect method such as a cuff compression method, a direct method such as an invasive method, or the like.

An example in which the blood pressure value at the notch point $P_{DN}$ in the blood pressure waveform of the test subject is about 90 mmHg is illustrated in FIG. 7. In this case, the waveform correction unit 22 multiplies a first-order differential absorbance at each time shown in the pulse wave waveform by a multiplication coefficient so that the pulse wave waveform value of the DN point $Q_{DN}$ detected in the pulse wave waveform after the first correction process becomes about 90 mmHg. Thereby, in the pulse wave waveform after the second correction process, the pulse wave waveform value of the DN point $Q_{DN}$ becomes about 90 mmHg.

Through the above-described process, the pulse wave waveform becomes a waveform corresponding to the blood pressure waveform. That is, the waveform correction unit 22 acquires a waveform corresponding to the blood pressure waveform. The waveform correction unit 22 may reduce an influence of the reflected wave within the artery by reconfiguring the waveform from a specific frequency component of the power spectrum obtained by performing, for example, a Fourier transform on the pulse wave waveform after the second correction process. Also, for example, physiological effects or the like in the living body may be reduced by reconfiguring the waveform after reducing or removing a low frequency component in the power spectrum. The waveform correction unit 22 outputs the waveform corresponding to the blood pressure waveform to the blood pressure calculation unit 23 and the display unit 24.

The blood pressure calculation unit 23 calculates a blood pressure value on the basis of the waveform corresponding to the blood pressure waveform output from the waveform correction unit 22 (see part (b) of FIG. 7). The blood pressure calculation unit 23 calculates the blood pressure value at each time point or calculates the blood pressure value at a time point set in advance. Also, the blood pressure calculation unit 23 may perform a process of integrating blood pressure values across a plurality of cycles or may calculate the blood pressure value as an average blood pressure in each cycle.

The display unit 24 displays at least one of the pulse wave waveform output from the pulse wave acquisition unit 21 (see part (b) of FIG. 3), the waveform corresponding to the blood pressure waveform output from the waveform correction unit 22 (see part (b) of FIG. 7), and the blood pressure value calculated by the blood pressure calculation unit 23 on, for example, a display of the computer 20 to be described below or the like. The display unit 24 may display a pulse wave waveform subjected to the Fourier transform or a waveform corresponding to the blood pressure waveform subjected to the Fourier transform. Also, a current blood pressure value may be displayed in real time or a maximum/minimum blood pressure value and an average blood pressure value per cycle may be displayed. Also, a pulse rate may be obtained by the pulse wave acquisition unit 21 and the pulse rate may be displayed simultaneously with the waveform or the blood pressure value.

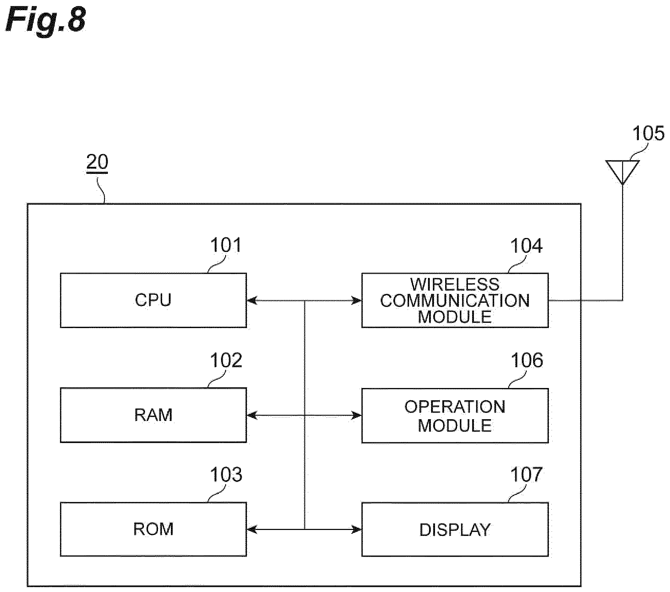

Next, a hardware configuration of the computer 20 will be described. FIG. 8 illustrates the hardware configuration of the computer 20 in FIG. 1. As illustrated in FIG. 8, the computer 20 physically includes a central processing unit (CPU) 101 which is a processor, a random access memory (RAM) 102 or a read only memory (ROM) 103 which is a recording medium, a wireless communication module 104, an antenna 105, an operation module 106, a display 107, and the like. These components are electrically connected to each other. Each function of the computer 20 described above causes the wireless communication module 104, the antenna 105, the operation module 106, the display 107, and the like to operate under the control of the CPU 101 by loading the blood pressure measurement program on hardware such as the CPU 101, the RAM 102, or the like, and is implemented by reading and writing data from and to the RAM 102. The above is the configuration of the computer 20 according to the present example

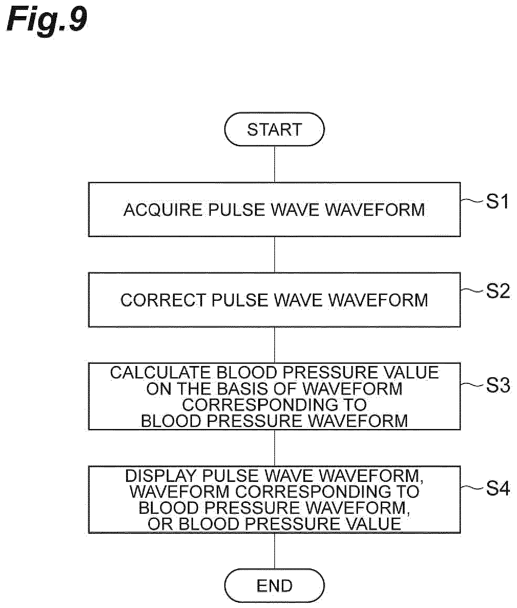

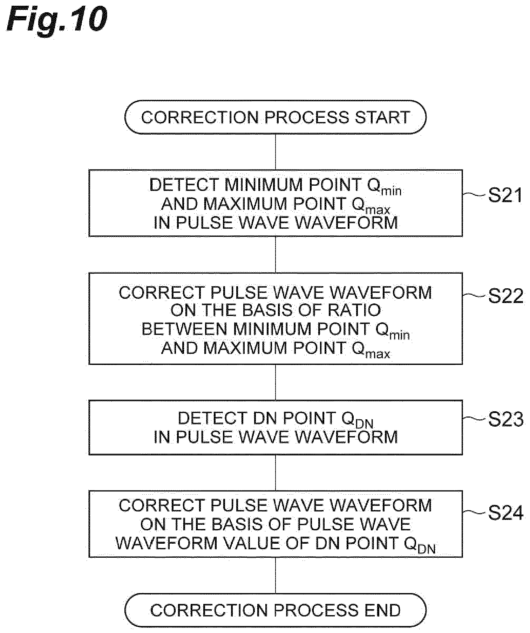

Next, a blood pressure measurement method which is an operation method of the computer 20 (a process to be executed by the computer 20) according to the present example will be described for each process with reference to the flowcharts of FIGS. 9 and 10. First, an example of the overall processing procedure will be described with reference to the flowchart of FIG. 9. FIG. 9 is a flowchart illustrating the blood pressure measurement method according to the first example.

In this case, as a premise of this process, the ratio between the diastolic blood pressure $P_{min}$ and the systolic blood pressure $P_{max}$ and the blood pressure value at the notch point $P_{DN}$ are set in advance in the computer 20. When the connection based on the wireless communication is established between the pulse wave measurement device 10 and the computer 20, the pulse wave acquisition unit 21 acquires a pulse wave waveform based on a volume pulse wave on the basis of information indicating the volume pulse wave transmitted from the pulse wave measurement device 10 (S1: pulse wave acquisition step). Subsequently, the acquired pulse wave waveform is corrected by the waveform correction unit 22 (S2: waveform correction step). Thereby, a waveform corresponding to the blood pressure waveform is acquired. A detailed processing procedure in the waveform correction step will be described below with reference to FIG. 10.

Subsequently, the blood pressure calculation unit 23 calculates a blood pressure value on the basis of the corrected pulse wave waveform, i.e., the waveform corresponding to the blood pressure waveform (S3: blood pressure calculation step). Subsequently, the display unit 24 displays at least one of the pulse wave waveform acquired in the pulse wave acquisition step S1, the waveform corresponding to the blood pressure waveform acquired in the waveform correction step S2 (a corrected pulse wave waveform), and the blood pressure value calculated in the blood pressure calculation step S3 on the display 107 of the computer 20 (S4: display step). Thus, the blood pressure measurement method ends. The blood pressure calculation step S3 and the display step S4 may not be included in the blood pressure measurement method. Also, the display step S4 may be performed between the pulse wave acquisition step S1 and the waveform correction step S2 or between the waveform correction step S2 and the blood pressure calculation step S3.

Next, a detailed processing procedure in the waveform correction step S2 will be described with reference to the flowchart of FIG. 10. FIG. 10 is a flowchart illustrating a detailed procedure in the waveform correction step S2 illustrated in FIG. 9.

When the processing of the waveform correction step S2 starts, the waveform correction unit 22 performs the processing of the following steps S21 to S24. First, the minimum point $Q_{min}$ and the maximum point $Q_{max}$ in the pulse wave waveform are detected (S21). Subsequently, a ratio between the minimum point $Q_{min}$ and the maximum point $Q_{max}$ is calculated and the pulse wave waveform is corrected on the basis of the ratio (S22). Specifically, an addition coefficient is added to the pulse wave waveform value at each time so that the ratio between the minimum point $Q_{min}$ and the maximum point $Q_{max}$ is the ratio between the diastolic blood pressure $P_{min}$ and the systolic blood pressure $P_{max}$ set in advance in the computer 20.

Subsequently, by performing the processing of S21 and S22, the DN point $Q_{DN}$ in the corrected pulse wave waveform is detected (S23: DN point detection step). Subsequently, the pulse wave waveform is corrected on the basis of the pulse wave waveform value at the DN point $Q_{DN}$ in the pulse wave waveform (S24). Specifically, the pulse wave waveform value at each time is multiplied by a multiplication coefficient so that the pulse wave waveform value at the DN point $Q_{DN}$ becomes the blood pressure value at the notch point $P_{DN}$ set in advance in the computer 20. As described above, the waveform correction process is completed by the waveform correction unit 22, and a waveform corresponding to the blood pressure waveform is acquired. The processing of S21 and S22 (first correction process) and the processing of S23 and S24 (second correction process) may be performed in reverse order. That is, with respect to the pulse wave waveform corrected on the basis of the pulse wave waveform value at the DN point $Q_{DN}$, the corrected pulse wave waveform may be further corrected so that the ratio between the minimum point $Q_{min}$ and the maximum point $Q_{max}$ of the corrected pulse wave waveform becomes a predetermined value. Also, the detection of the minimum point $Q_{min}$ and the maximum point $Q_{max}$ in S21 and the detection of the DN point $Q_{DN}$ in S23 may be performed collectively before the processing of S22 and S24.

As described above, in the computer 20 and the blood pressure measurement method using the computer 20 according to the present example, the DN point $Q_{DN}$ corresponding to the notch point $P_{DN}$ in a blood pressure waveform is detected in a pulse wave waveform and the pulse wave waveform is corrected so that a pulse wave waveform value of the DN point $Q_{DN}$ becomes a predetermined blood pressure value. Consequently, using, for example, the blood pressure value of the notch point $P_{DN}$ in the blood pressure waveform measured in advance as a reference value, it is possible to acquire a waveform corresponding to the blood pressure waveform by correcting the pulse wave waveform so that the pulse wave waveform value of the DN point $Q_{DN}$ in the pulse wave waveform becomes the reference value. Thereby, a series of time-varying blood pressure values can be accurately and easily obtained.

According to the present example, the pulse wave waveform acquired in the pulse wave acquisition step S1 is a waveform obtained by differentiating the volume pulse wave with respect to time. Thus, the DN point $Q_{DN}$ becomes clearer in the waveform, so that the DN point $Q_{DN}$ can be easily detected.

When the pulse wave waveform acquired in the pulse wave acquisition step S1 is the waveform of the volume pulse wave, it is possible to correct the waveform of the volume pulse wave so that the pulse wave waveform value of the DN point $Q_{DN}$ becomes the reference value in the waveform of the volume pulse wave by using the blood pressure value at the notch point $P_{DN}$ as the reference value. Thereby, a waveform corresponding to the blood pressure waveform can be acquired on the basis of the waveform of the volume pulse wave and a series of time-varying blood pressure values can be accurately and easily obtained.

According to the present example, in the blood pressure calculation step S3, the blood pressure value at each time point can be obtained by calculating the blood pressure value on the basis of the waveform corresponding to the blood pressure waveform acquired in the waveform correction step S2.

According to the present example, in the waveform correction step S2, a pulse wave waveform to be subjected to correction based on the pulse wave waveform value of the DN point $Q_{DN}$ is a pulse wave waveform corrected on the basis of the ratio between the minimum point $Q_{min}$ and the maximum point $Q_{max}$ in the pulse wave waveform by using the ratio between the diastolic blood pressure $P_{min}$ and the systolic blood pressure $P_{max}$ measured in advance as the reference value. Consequently, because the DN point becomes clearer in the pulse wave waveform, the DN point can be easily detected and the waveform corresponding to the blood pressure waveform can be acquired more accurately.

Also, even when the processing of S21 and S22 (first correction process) and the processing of S23 and S24 (second correction process) are performed in the reverse order, the waveform corresponding to the blood pressure waveform can be acquired more accurately because the ratio between the minimum point $Q_{min}$ and the maximum point $Q_{max}$ in the pulse wave waveform corrected on the basis of the pulse wave waveform value of the DN point $Q_{DN}$ is corrected to be the reference value by using the ratio between the diastolic blood pressure $P_{min}$ and systolic blood pressure $P_{max}$ measured in advance as the reference value.

According to the present example, in the display step S4, at least one of the pulse wave waveform acquired in the pulse wave acquisition step S1, the waveform corresponding to the blood pressure waveform acquired by correction in the waveform correction step S2, and the blood pressure value calculated in the blood pressure calculation step S3 is displayed. Thus, it is possible to visualize information of the pulse wave waveform, the waveform corresponding to the blood pressure waveform, or the blood pressure value, and allow a measurer to visually confirm the information.

(Second example)

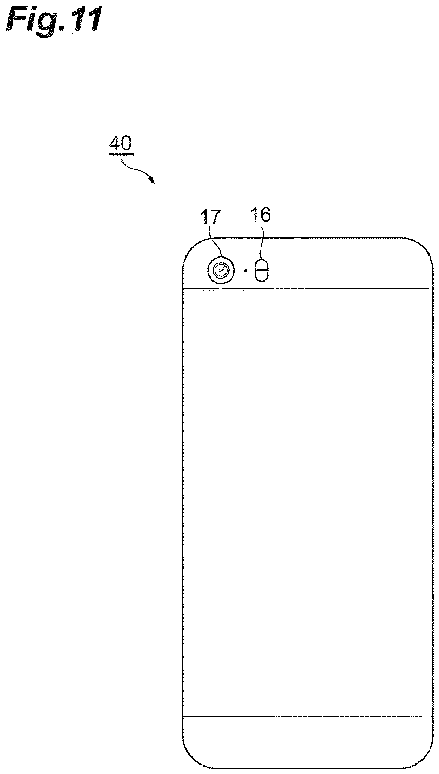

Next, a configuration of a blood pressure measurement device according to the second example will be described with reference to FIG. 11. FIG. 11 is a schematic configuration diagram illustrating the blood pressure measurement device according to the second example. As illustrated in FIG. 11, in the present example, the communication terminal 40 such as a smartphone functions as a blood pressure measurement device. A communication terminal such as a smartphone is included in a computer having a processor, a storage medium, or the like. The communication terminal 40 has a function similar to that of the computer 20 according to the first embodiment. That is, similar to the computer 20, the communication terminal 40 has functions as a pulse wave acquisition unit 21, a waveform correction unit 22, a blood pressure calculation unit 23, and a display unit 24.

The difference between the communication terminal 40 and the computer 20 is that the communication terminal 40 also has a function as the pulse wave measurement device 10 according to the first embodiment. That is, the pulse wave acquisition unit 21 of the communication terminal 40 includes a light source (light irradiation device) 16 that radiates light to an inside of the living body H as a test subject and a photodetector 17 that detects light radiated from the light source 16 and transmitted through the inside of the living body H. The light source 16 is, for example, a flash lamp of the communication terminal 40. The photodetector 17 is, for example, a camera of the communication terminal 40. In addition to the flash lamp and the camera, the communication terminal 40 may have a light source 16 and a photodetector 17 dedicated for pulse wave measurement. A tablet computer or the like may be also included in a computer having a processor, a storage medium, and the like, and a tablet computer or the like may be used instead of the communication terminal 40.

In the present embodiment, in a state in which a surface of the living body H (e.g., a finger) serving as a test subject is placed on both the light source 16 and the photodetector 17 of the communication terminal 40, the pulse wave acquisition unit 21 radiates light from the light source 16 from the surface of the living body H toward the inside thereof. Then, the pulse wave acquisition unit 21 detects reflected light from the living body H with the photodetector 17. Thereby, the pulse wave acquisition unit 21 acquires a volume pulse wave. Subsequently, the pulse wave acquisition unit 21 acquires a pulse wave waveform as in the first example on the basis of its own acquired volume pulse wave. Then, as in the first example, by correcting the pulse wave waveform, a waveform corresponding to the blood pressure waveform is acquired. Thereby, also in the present example, a series of time-varying blood pressure values can be accurately and easily obtained.

Further, according to the present example, the pulse wave acquisition unit 21 in the communication terminal 40 includes the light source 16 and the photodetector 17. Consequently, by detecting the light radiated from the light source 16 in the pulse wave acquisition unit 21 and transmitted through the inside of the living body H with the photodetector 17 in the pulse wave acquisition unit 21, it is possible to easily acquire a pulse wave waveform without providing the pulse wave measurement device 10 separately from the communication terminal 40 which is a blood pressure measurement device.

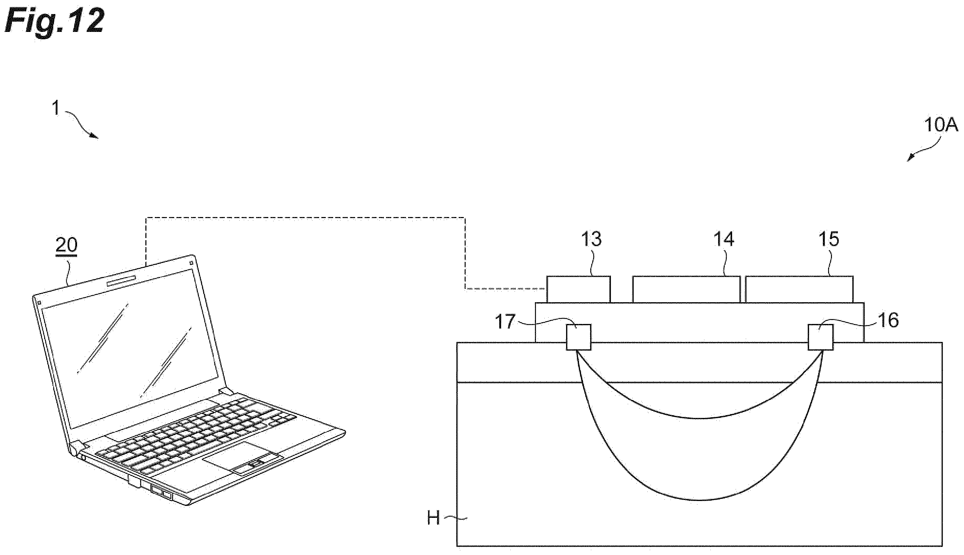

In a modified example illustrated in FIG 12, a pulse wave measurement device 10A in which a measurement instrument and a probe are integrated may be used instead of the pulse wave measurement device 10 according to the first embodiment. The pulse wave measurement device 10A is attached to a surface of a living body H and includes, for example, a communication unit 13, a processing unit 14, a power supply unit 15, a light source 16, and a photodetector 17. Also in the blood pressure measurement device according to this modified example, it is possible to accurately and easily obtain a series of time-varying blood pressure values as in the above-described example.

The computer 20 is a blood pressure measurement device in the above example, but a configuration including the pulse wave measurement device 10 or 10A may be a blood pressure measurement device. Also, the surface of the living body H serving as a test subject may be other than a palm or a finger and may be a forehead, an upper arm, a neck, an earlobe or the like.

In the blood pressure measurement method according to the above, by using the blood pressure value at the dicrotic notch point as a reference value, the waveform of the volume pulse wave can be corrected so that the time waveform value of the DN point becomes the reference value in the waveform of the volume pulse wave. Thereby, a waveform corresponding to the blood pressure waveform can be acquired on the basis of the waveform of the volume pulse wave and a series of time-varying blood pressure values can be accurately and easily obtained.

In the blood pressure measurement method according to the above, because the DN point becomes clearer in the waveform obtained by differentiating the volume pulse wave with respect to time, the DN point can be easily detected.

In the blood pressure measurement method according to the above, because the DN point becomes clearer in the volume pulse wave waveform, it is possible to easily detect the DN point.

In the blood pressure measurement method according to the above, the corrected time waveform can be further corrected so that the ratio between the minimum point and the maximum point in the time waveform corrected on the basis of the DN point becomes a reference value by using, for example, the ratio between the diastolic blood pressure and the systolic blood pressure measured in advance as the reference value. Thereby, a waveform corresponding to the blood pressure waveform can be acquired more accurately.

The blood pressure measurement method according to the above, because the time waveform corrected in the waveform correction step corresponds to a blood pressure waveform, the blood pressure value at each time point can be obtained by calculating the blood pressure value based on the waveform corresponding to the blood pressure waveform.

The blood pressure measurement method according to the above, it is possible to visualize information of the time waveform, the corrected time waveform, or the blood pressure value, and allow a measurer to visually confirm the information.

In the blood pressure measurement device according to another aspect, the pulse wave acquisition unit may include an irradiation device configured to radiate light inside a living body; and a photodetector configured to detect the light transmitted through an inside of the living body. In this case, by detecting the light radiated from the irradiation device in the pulse wave acquisition unit and transmitted through the inside of the living body with the photodetector in the pulse wave acquisition unit, it is possible to easily acquire the time waveform without providing the pulse wave measurement device separately from the blood pressure measurement device.

Also, for example, a pulse wave waveform spectrum in the computer 20 is generated by performing a Fourier transform on a pulse wave waveform and a ratio $P_{Tmax}:P_{Tmin}$ between maximum and minimum blood pressures corresponding to a ratio between a diastolic blood pressure $P_{min}$ and a systolic blood pressure $P_{max}$ in the blood pressure waveform of the test subject may be calculated on the basis of the pulse wave waveform spectrum. In detail, the ratio $P_{Tmax}:P_{Tmin}$ between the maximum and minimum blood pressures in the test subject is calculated on the basis of a pulse wave waveform spectrum $P'_F$ having a frequency equal to or higher than a frequency corresponding to the pulse of the test subject in the pulse wave waveform spectrum $P'_F$. More specifically, the ratio $P_{Tmax}:P_{Tmin}$ between the maximum and minimum blood pressures is calculated on the basis of the following Equation (1). As a result of repeated intensive research by the present inventors, Equation (1) shows a newly found statistically significant correspondence relationship. Details of the correspondence relationship and Equation (1) will be described below. The pulse wave waveform spectrum may be generated from the pulse wave waveform corrected on the basis of the pulse wave waveform value at the DN point $Q_{DN}$.

[Math. 1]

$$P_{T\min} : P_{T\max} = \left| P'_F\left(f_1\right) \right| : \sum_{n=1}^{N} \left| P'_F\left(f_n\right) \right| \qquad \cdots (1)$$

Here, in Equation (1), n represents a positive integer, $f_1$ represents a frequency corresponding to a pulse, and $f_n$ represents a frequency which is n times the frequency corresponding to the pulse.

Hereinafter, a wave with the frequency $f_1$ corresponding to the pulse in the pulse wave waveform spectrum $P'_F$ is defined as a first harmonic wave and a wave with the frequency $f_n$ which is n times the frequency $f_1$ of the first harmonic wave is defined as an $n^{th}$ harmonic wave. The frequency $f_1$ corresponding to the pulse is a frequency range corresponding to a pulse that the human body can cope with, and is, for example, about 0.5 Hz to 3.7 Hz. The frequency $f_1$ corresponding to the pulse fluctuates within the frequency range (about 0.5 Hz to 3.7 Hz) corresponding to the pulse that the human body can cope with according to changes in the living body, and the frequency $f_n$ also changes in accordance therewith. In the above-described Equation (1), $P'_F(f_1)$ represents a spectral intensity of the first harmonic wave, and $P'_F(f_n)$ represents a spectral intensity of the $n^{th}$ harmonic wave. The spectral intensity of the first harmonic wave is, for example, a peak value of the spectral intensity of the first harmonic wave, and the spectral intensity of the $n^{th}$ harmonic wave is, for example, a peak value of the spectral intensity of the $n^{th}$ harmonic wave.

The ratio $P_{Tmax}:P_{Tmin}$ between the maximum and minimum blood pressures is calculated by calculating a ratio between a sum of peak values of spectral intensities of the first harmonic wave or more in the pulse wave waveform spectrum $P'_F$ and a peak value of a spectral intensity of the first harmonic wave in the pulse wave waveform spectrum $P'_F$ on the basis of the above-described Equation (1). When the ratio $P_{Tmax}:P_{Tmin}$ between the maximum and minimum blood pressures is calculated according to the above-described Equation (1), for example, N = 3 may be used. That is, at least peak values of spectral intensities from the first harmonic wave to the third harmonic wave may be used. Also, N may be 6. That is, peak values of the spectral intensities from the first harmonic wave to the sixth harmonic wave may be used. More specifically, because a component having a frequency higher than 30 Hz in the pulse wave waveform spectrum $P'_F$ is noise, a peak value of the spectral intensity of 30 Hz or less may be used so that the noise is not reflected in the calculation result, and preferably, a peak value of a spectral intensity of 20 Hz or less is used.

Also, the ratio $P_{Tmax}:P_{Tmin}$ between the maximum and minimum blood pressures may be calculated on the basis of a sum of intensities of relative blood pressure waveform spectra of a first harmonic wave group or more. Specifically, the ratio $P_{Tmax}:P_{Tmin}$ between the maximum and minimum blood pressures may be calculated on the basis of the following Equation (2). As a result of repeated intensive research by the present inventors, Equation (2) shows a newly found statistically significant correspondence relationship. Details of the correspondence relationship and Equation (2) will be described below.

[Math. 2]

$$P_{T\min} : P_{T\max} = \int_{-d}^{d}\left|P'_F\left(f_1 + \frac{f}{2}\right)\right|df : \sum_{n=1}^{N}\int_{-d}^{d}\left|P'_F\left(f_n + \frac{f}{2}\right)\right|df \qquad \cdots(2)$$

Here, in Equation (2), n represents a positive integer, $f_1$ represents a frequency corresponding to a pulse, and $f_n$ represents a frequency which is n times the frequency corresponding to the pulse.

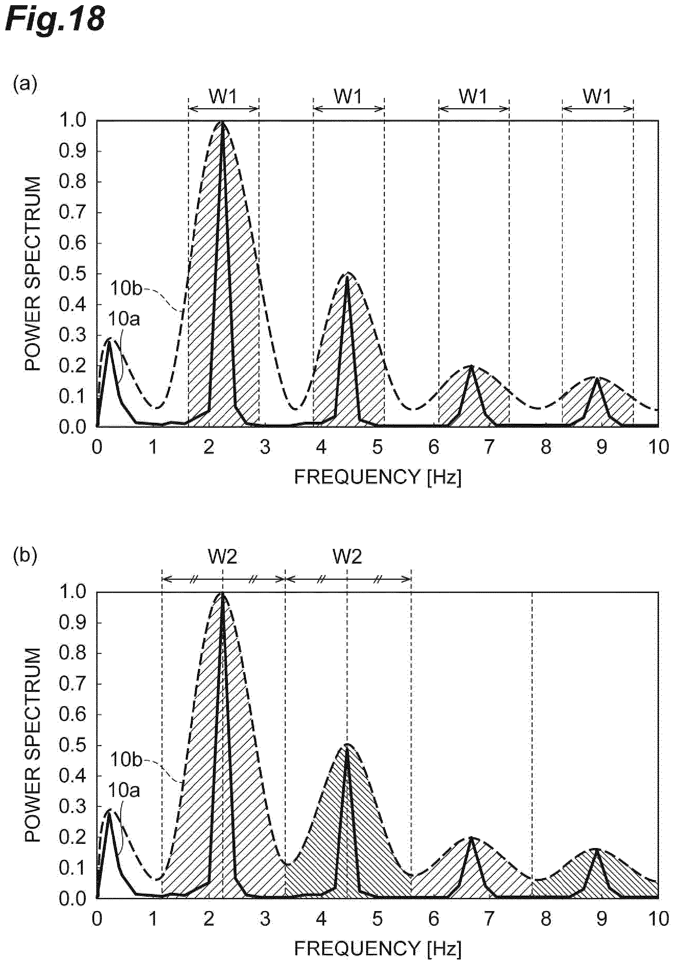

Hereinafter, a wave group having a frequency $f_1$ corresponding to a pulse and having a frequency within a predetermined range of a frequency $f_1$ corresponding to a pulse is set as a first harmonic wave group. More specifically, the first harmonic wave group is, for example, a spectrum in a range of a predetermined effective width centered on the peak value of the spectral intensity of the first harmonic wave. The spectral intensity of the first harmonic wave group is, for example, an integral value of spectral intensities at a predetermined effective width. Also, a wave group having a frequency $f_n$ which is n times the frequency $f_1$ corresponding to the pulse and having a frequency in a predetermined range of the frequency $f_n$ which is n times the frequency $f_1$ is set as an $n^{th}$ harmonic wave group. More specifically, the $n^{th}$ harmonic wave group is, for example, a spectrum in a range of a predetermined effective width centered on the peak value of the spectral intensity of the $n^{th}$ harmonic wave. The spectral intensity of the $n^{th}$ harmonic wave group is, for example, an integral value of spectral intensities at a predetermined effective width. A specific example of the predetermined effective width will be described below with reference to FIG. 18.

The ratio $P_{Tmax}:P_{Tmin}$ between the maximum and minimum blood pressures is calculated by calculating a ratio between a sum of peak values of spectral intensities of the first harmonic wave group or more in the pulse wave waveform spectrum $P'_F$ and a peak value of a spectral intensity of the first harmonic wave group in the pulse wave waveform spectrum $P'_F$ on the basis of the above-described Equation (2). In the above-described Equation (2), N may be set to 3. That is, at least an integral value of spectral intensities from the first harmonic wave group to the third harmonic wave group may be used. Also, N may be 6. That is, an integral value of spectral intensities from the first harmonic wave group to the sixth harmonic wave group may be used. More specifically, because a component having a frequency higher than 30 Hz in the pulse wave waveform spectrum $P'_F$ is noise, an integral value of spectral intensities of 30 Hz or less may be used so that the noise is not reflected in the calculation result, and preferably, an integral value of spectral intensities of 20 Hz or less is used.

Next, a correspondence relationship indicating the above-described Equation (1) found by the present inventors will be described in detail.

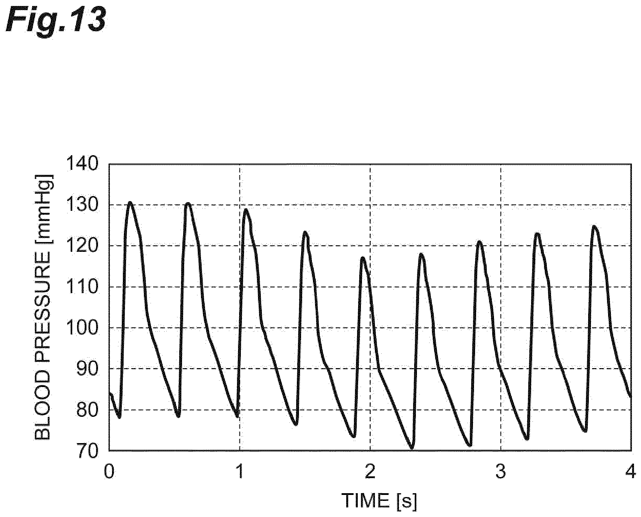

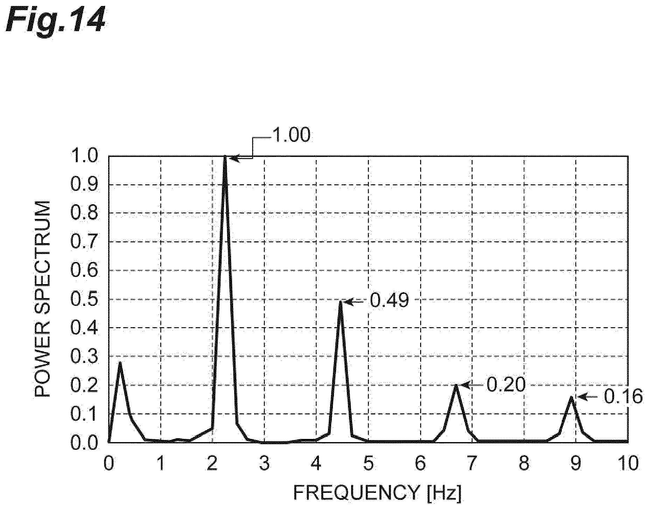

FIG. 13 is a graph illustrating a blood pressure waveform measured by an invasive catheter sphygmomanometer. The horizontal axis of FIG. 13 represents time [s] and the vertical axis of FIG. 13 represents a blood pressure [mmHg]. In the graph illustrated in FIG. 13, a maximum blood pressure value $P_{Tmax}$ is about 130 mmHg and a minimum blood pressure value $P_{Tmin}$ is about 70 mmHg. Consequently, the ratio between the maximum blood pressure value $P_{Tmax}$ and the minimum blood pressure value $P_{Tmin}$, i.e., the ratio between the maximum and minimum blood pressures, is about 1.86. The blood pressure waveform mainly includes a first harmonic wave (main wave) with the frequency $f_1$ corresponding to the pulse and an $n^{th}$ harmonic wave with the frequency $f_n$ larger than the frequency $f_1$. When the blood pressure waveform illustrated in FIG. 13 is subjected to a Fourier transform, a power spectrum as illustrated in FIG. 14 is obtained.

FIG. 14 is a graph illustrating a power spectrum obtained by performing a Fourier transform on a blood pressure waveform illustrated in FIG. 13. The power spectrum is normalized by the spectral intensity of the first harmonic wave, the horizontal axis of FIG 14 represents a frequency [Hz], and the vertical axis of FIG. 14 represents a spectral intensity. As a result of repeated intensive research, the present inventors newly found that a ratio between a sum of spectral intensities of the $n^{th}$ harmonic wave which is equal to or greater than the first harmonic wave and a spectral intensity of the first harmonic wave (hereinafter also referred to as a "spectral intensity-based ratio") is substantially equal to the ratio between the maximum and minimum blood pressures obtained from the blood pressure waveform illustrated in FIG. 13 in the power spectrum illustrated in FIG. 14. Specifically, in the power spectrum illustrated in FIG. 14, the sum of the spectral intensities of the $n^{th}$ harmonic wave which is equal to or greater than the first harmonic wave is 1.00+0.49+0.20+0.16 = 1.85. Consequently, the ratio between the sum of the spectral intensities of the $n^{th}$ harmonic wave which is equal to or greater than the first harmonic wave and the spectral intensity of the first harmonic wave is 1.85 and is substantially equal to about 1.86 which is the ratio between the maximum blood pressure value $P_{Tmax}$ and the minimum blood pressure value $P_{Tmin}$ in the true blood pressure waveform. This correspondence relationship can be represented by the above-described Equation (1).

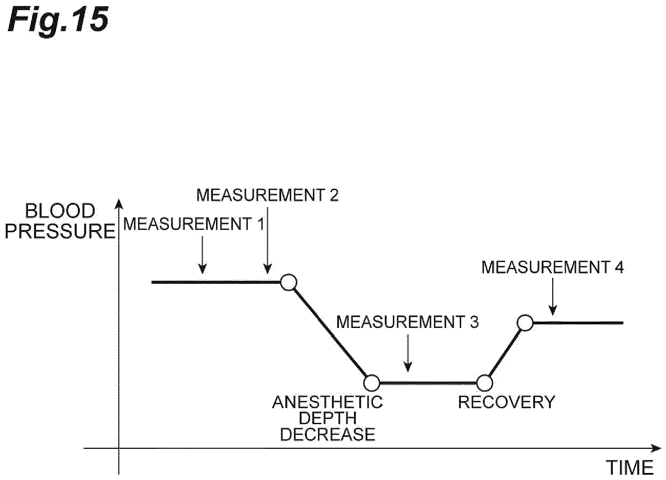

The present inventors confirmed that the correspondence relationship shown in the above-described Equation (1) is statistically significant through the following experiment. The present inventors continuously measured a blood pressure waveform indicating a change in a blood pressure of a cynomolgus monkey while changing the blood pressure by giving isoflurane anesthetic agents of different concentrations to the cynomolgus monkey in a state in which an invasive blood pressure monitor was installed in the artery of the foot of the cynomolgus monkey. FIG. 15 is a graph illustrating a change in a blood pressure of a cynomolgus monkey due to an anesthetic agent. The horizontal axis of FIG. 15 represents time and the vertical axis of FIG. 15 represents a blood pressure.

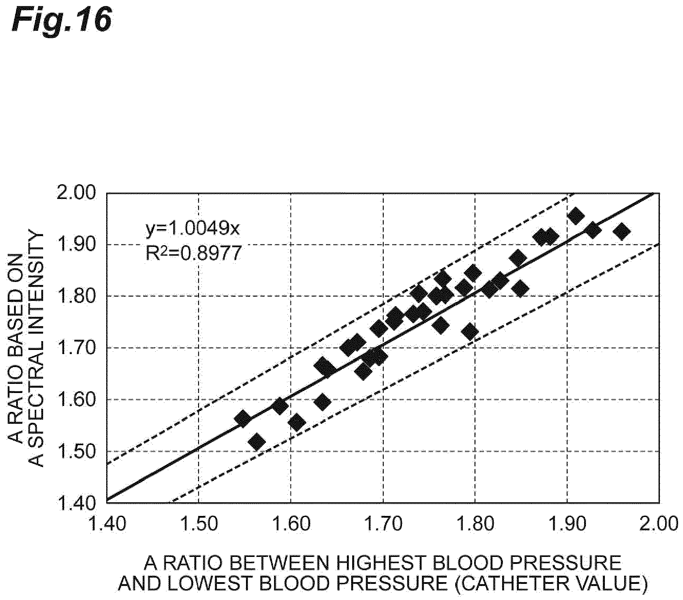

Then, a correlation relationship was confirmed by extracting a plurality of pieces of data in different time periods of the measured blood pressure waveform and plotting a relationship between a ratio between the maximum blood pressure and the minimum blood pressure obtained from the extracted data and a spectral intensity-based ratio obtained by performing a Fourier transform on the blood pressure waveform as illustrated in a graph of FIG. 16. The horizontal axis of FIG. 16 represents the ratio between the maximum and minimum blood pressures obtained by experiments performed on cynomolgus monkeys and the vertical axis of FIG. 16 represents the ratio based on the spectral intensity obtained by performing a Fourier transform on the blood pressure waveform. As illustrated in FIG. 16, it was confirmed that the spectral intensity-based ratio obtained by performing the Fourier transform on the blood pressure waveform falls within a range of ±5% of the ratio between the maximum blood pressure and the minimum blood pressure obtained by the experiment performed on the cynomolgus monkeys.

A correspondence relationship shown in the above-described Equation (1) was shown to be statistically significant from the above.

The accuracy of the relationship shown in the above-described Equation (1) depends on the frequency resolution of the Fourier transform. When one pulse wave is considered, there is no wave with a frequency other than an integer multiple of the frequency corresponding to the pulse ideally. However, when a plurality of pulse waves are considered, a wave with a frequency other than an integer multiple of a frequency corresponding to a pulse may be included according to living body fluctuation.

The frequency resolution of the Fourier transform depends on a length of the time waveform before conversion in principle, but the spectrum of the time waveform cannot be completely separated for each frequency because the time waveform actually measured is a finite length. The spectra of integer-multiple waves include spectra of waves around the integer-multiple waves other than the integer-multiple waves. When the frequency resolution is higher, waves other than the integer-multiple waves can be eliminated and the accuracy of the relationship shown in the above-described Equation (1) is improved. Conversely, when the frequency resolution is lower, there is more influence from waves other than the integer-multiple waves and the accuracy decreases. Although there is a difference in accuracy depending on the frequency resolution of the Fourier transform, the correspondence relationship expressed by the above-described Equation (1) remains significant statistically.

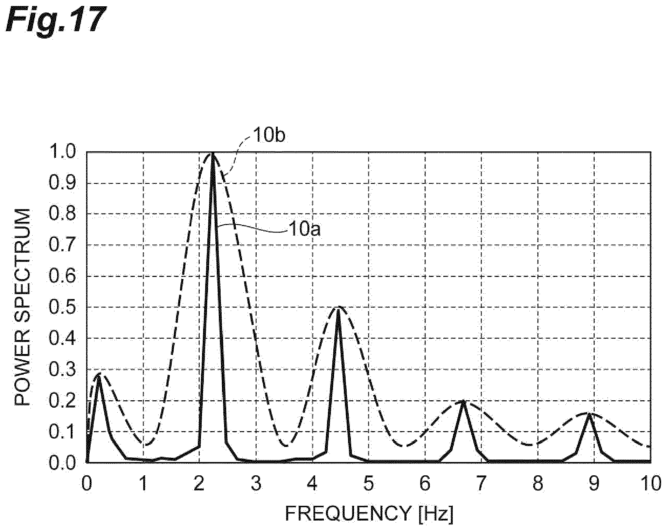

FIG. 17 is a graph illustrating a spread of a power spectrum of a blood pressure waveform due to living body fluctuation. The horizontal axis of FIG. 17 represents a frequency [Hz], and the vertical axis of FIG. 17 represents a spectral intensity. A graph 10a of FIG. 17 shows a power spectrum of an ideal blood pressure waveform ignoring living body fluctuation and a graph 10b of FIG. 17 shows a power spectrum of a blood pressure waveform including waves other than integer-multiple waves due to living body fluctuation. The graph 10b showing the power spectrum of the blood pressure waveform including waves other than integer-multiple waves due to living body fluctuation has wider peaks than the graph 10a showing the power spectrum of the ideal blood pressure waveform for which the living body fluctuation is ignored.

The present inventors found that a ratio between the sum of the spectral intensities of the $n^{th}$ harmonic wave group equal to or greater than the first harmonic wave group and the spectral intensity of the first harmonic wave group is substantially equal to the ratio between the maximum blood pressure value $P_{Tmax}$ and the minimum blood pressure value $P_{Tmin}$ in a power spectrum of a blood pressure waveform in which waves other than integer-multiple waves are included due to living body fluctuation as described above. That is, a correspondence relationship represented by the above-described Equation (2) was found to be satisfied.

By establishing the correspondence relationship shown in the above-described Equation (2), the ratio between the maximum and minimum blood pressures can be obtained from an integral value of the spectral intensity at each predetermined effective width including each peak value as described above. Here, the predetermined effective width may be, for example, a frequency width W1 which is a value at half of the peak value of the spectral intensity of the $n^{th}$ harmonic wave as illustrated in part (a) of FIG. 18 or may be, for example, a frequency width W2 demarcated by centers between frequencies of adjacent $n^{th}$ harmonic waves as illustrated in part (b) of FIG. 18. The optimum frequency resolution or the effective width of the spectrum group may be appropriately set as in consideration of device characteristics of a blood pressure ratio calculation device or the living body fluctuation or the like.

Also, a blood pressure measurement program according to one example includes a main module, a pulse wave acquisition module, a DN point detection module, and a waveform correction module.

The main module is a part for controlling a blood pressure measurement process in an integrated manner. The pulse wave acquisition module is a part that performs a pulse wave acquisition process. The function realized by running the pulse wave acquisition module is similar to the above-described function of the pulse wave acquisition unit 21 of the computer 20. The DN point detection module performs a DN point detection process, and the waveform correction module performs a waveform correction process. The function realized by running the DN point detection module and the waveform correction module is similar to the function of the waveform correction unit 22 of the computer 20 described above.

The blood pressure measurement program is provided by, for example, a recording medium or a semiconductor memory such as a CD-ROM, DVD, or ROM. In addition, the blood pressure measurement program may be provided as a computer data signal superimposed on a carrier wave via a network.

Also, an aspect of the present invention is a program for causing a computer to execute blood pressure measurement for acquiring a change in a blood pressure with respect to time.

**Industrial Applicability**

According to an aspect of the present invention, it is possible to accurately and easily obtain a series of time-varying blood pressure values using a blood pressure measuring method, a blood pressure measurement device, a blood pressure measuring program, and a computer-readable storage medium as application forms.

**Reference Signs List**

16 Light source (light irradiation device)
17 Photodetector
20 Computer (blood pressure measurement device)
21 Pulse wave acquisition unit
22 Waveform correction unit
23 Blood pressure calculation unit
40 Communication terminal (blood pressure measurement device)
S1 Pulse wave acquisition step
S2 Waveform correction step
S23 DN point detection step
S3 Blood pressure calculation step
S4 Display step
$P_{min}$ Diastolic blood pressure
$P_{max}$ Systolic blood pressure
$P_{DN}$ Dicrotic notch point
$Q_{min}$ Minimum point
$Q_{max}$ Maximum point
$Q_{DN}$ DN point
H Living body.

**Claims**

1. A blood pressure measurement method of acquiring a change in a blood pressure with respect to time, the blood pressure measurement method comprising:

   a pulse wave acquisition step of acquiring a time waveform based on a volume pulse wave, wherein the time waveform is a waveform obtained by adding an addition coefficient to a volume pulse wave waveform value at each time, the volume pulse wave waveform being a waveform of the volume pulse wave or a waveform obtained by differentiating the volume pulse wave with respect to time, so that a ratio between a minimum value point and a maximum value point of the volume pulse wave waveform becomes a predetermined value that is set in advance on the basis of a ratio between a diastolic blood pressure and a systolic blood pressure in a blood pressure waveform of a test subject calculated or measured in advance;
   a DN point detection step of detecting a DN point, which corresponds to a dicrotic notch point in the blood pressure waveform of the test subject indicating the change in the blood pressure with respect to time, in the time waveform; and
   a waveform correction step of correcting the time waveform by multiplying the time waveform by a multiplication coefficient so that a time waveform value at the DN point becomes a predetermined blood pressure value that is set in advance on the basis of the blood pressure value at the dicrotic notch point in the blood pressure waveform of the test subject calculated or measured in advance; and
   acquiring the change in the blood pressure with respect to time based on the corrected time waveform.

2. A blood pressure measurement method of acquiring a change in a blood pressure with respect to time, the blood pressure measurement method comprising:

   a pulse wave acquisition step of acquiring a time waveform based on a volume pulse wave, wherein the time waveform is a waveform of the volume pulse wave or a waveform obtained by differentiating the volume pulse wave with respect to time;
   a DN point detection step of detecting a DN point, which corresponds to a dicrotic notch point in a blood pressure waveform of a test subject indicating the change in the blood pressure with respect to time, in the time waveform; and
   a waveform correction step of correcting the time waveform by multiplying the time waveform by a multiplication coefficient so that a time waveform value at the DN point becomes a predetermined blood pressure value that is set in advance on the basis of the blood pressure value at the dicrotic notch point in the blood pressure waveform of the test subject calculated or measured in advance, wherein the corrected time waveform is further corrected by adding an addition coefficient to a corrected time waveform value at each time, so that a ratio between a minimum value point and a maximum value point of the corrected time waveform becomes a predetermined value that is set in advance on the basis of a ratio between a diastolic blood pressure and a systolic blood pressure in the blood pressure waveform of the test subject calculated or measured in advance; and
   acquiring the change in the blood pressure with respect to time based on the corrected time waveform.

3. The blood pressure measurement method according to claim 1 or 2, wherein the time waveform comprises a waveform of the volume pulse wave.

4. The blood pressure measurement method according to claim 1 or 2, wherein the time waveform comprises a waveform obtained by differentiating the volume pulse wave with respect to time.

5. The blood pressure measurement method according to any one of claims 1 to 4, further comprising:
   a blood pressure calculation step of calculating a blood pressure value on the basis of the corrected time waveform.

6. The blood pressure measurement method according to claim 5, further comprising:
   a display step of displaying at least one of the time waveform, the corrected time waveform, and the blood pressure value.

7. A blood pressure measurement device configured to acquire a change in a blood pressure with respect to time, the blood pressure measurement device comprising:

   a pulse wave acquisition unit configured to acquire a time waveform based on a volume pulse wave, wherein the time waveform is a waveform obtained by adding an addition coefficient to a volume pulse wave waveform value at each time, the volume pulse wave waveform being a waveform of the volume pulse wave or a waveform obtained by differentiating the volume pulse wave with respect to time, so that a ratio between a minimum value point and a maximum value point of the volume pulse wave waveform becomes a predetermined value that is set in advance on the basis of a ratio between a diastolic blood pressure and a systolic blood pressure in a blood pressure waveform of a test subject calculated or measured in advance; and

a waveform correction unit configured to detect a DN point, which corresponds to a dicrotic notch point in the blood pressure waveform of the test subject indicating the change in the blood pressure with respect to time, in the time waveform and correct the time waveform by multiplying the time waveform by a multiplication coefficient so that a time waveform value at the DN point becomes a predetermined blood pressure value that is set in advance on the basis of the blood pressure value at the dicrotic notch point in the blood pressure waveform of the test subject calculated or measured in advance and acquire the change in the blood pressure with respect to time based on the corrected time waveform.

8. A blood pressure measurement device configured to acquire a change in a blood pressure with respect to time, the blood pressure measurement device comprising:

a pulse wave acquisition unit configured to acquire a time waveform based on a volume pulse wave, wherein the time waveform is a waveform of the volume pulse wave or a waveform obtained by differentiating the volume pulse wave with respect to time; and

a waveform correction unit configured to detect a DN point, which corresponds to a dicrotic notch point in a blood pressure waveform of a test subject indicating the change in the blood pressure with respect to time, in the time waveform and correct the time waveform by multiplying the time waveform by a multiplication coefficient so that a time waveform value at the DN point becomes a predetermined blood pressure value that is set in advance on the basis of the blood pressure value at the dicrotic notch point in the blood pressure waveform of the test subject calculated or measured in advance, wherein the corrected time waveform is further corrected by adding an addition coefficient to a corrected time waveform value at each time, so that a ratio between a minimum value point and a maximum value point of the corrected time waveform becomes a predetermined value that is set in advance on the basis of a ratio between a diastolic blood pressure and a systolic blood pressure in the blood pressure waveform of the test subject calculated or measured in advance and acquire the change in the blood pressure with respect to time, based on the corrected time waveform.

9. The blood pressure measurement device according to claim 7 or 8, wherein the time waveform comprises a waveform of the volume pulse wave.

10. The blood pressure measurement device according to claim 7 or 8, wherein the time waveform comprises the waveform obtained by differentiating a volume pulse wave with respect to time.

11. The blood pressure measurement device according to claim 7 or 8, further comprising:
a blood pressure calculation unit configured to calculate a blood pressure value on the basis of the corrected time waveform.

12. The blood pressure measurement device according to claim 11, further comprising:
a display unit configured to display at least one of the time waveform, the corrected time waveform, and the blood pressure value.

13. The blood pressure measurement device according to any one of claims 7 to 12, wherein the pulse wave acquisition unit comprises:

an irradiation device configured to radiate light inside a living body; and
a photodetector configured to detect the light transmitted through an inside of the living body.

14. A blood pressure measurement program comprising instructions which, when the program is executed by a computer, cause the computer to execute the steps of the method of any of claims 1 - 6.

15. A computer-readable storage medium comprising instructions which, when executed by a computer, cause the computer to carry out the method of any of claims 1 - 6.

**Patentansprüche**

1. Blutdruckmessungsverfahren zum Erfassen einer zeitlichen Änderung eines Blutdrucks, wobei das Blutdruckmessungsverfahren umfasst:

einen Pulswellenerfassungsschritt zum Erfassen einer Zeitwellenform basierend auf einer Volumenpulswelle, wobei die Zeitwellenform eine Wellenform ist, die durch Addieren eines Additionskoeffizienten zu einem Volumenpulswellenformwert zu jeder Zeit erhalten wird, wobei die Volumenpulswellenform eine Wellenform der Volumenpulswelle oder eine Wellenform ist, die durch Differenzieren der Volumenpulswelle in Bezug auf die Zeit erhalten wird, so dass ein Verhältnis zwischen einem Minimalwertpunkt und einem Maximalwertpunkt der Volumenpulswellenform zu einem vorbestimmten Wert wird, der im Voraus auf Basis eines im Voraus berechneten oder gemessenen Verhältnisses zwischen einem diastolischen Blutdruck und einem systolischen Blutdruck in einer Blutdruckwellenform einer Testperson festgelegt wird;
einen DN-Punkt-Erkennungsschritt zum Erkennen eines DN-Punktes, der einem dikrotischen Kerbenpunkt in der Blutdruckwellenform der Testperson entspricht, der die zeitliche Änderung des Blutdrucks angibt, in der Zeitwellenform; und
einen Wellenformkorrekturschritt zum Korrigieren der Zeitwellenform durch Multiplizieren der Zeitwellenform mit einem Multiplikationskoeffizienten, so dass ein Zeitwellenformwert am DN-Punkt zu einem vorbestimmten Blutdruckwert wird, der im Voraus auf Basis des Blutdruckwertes am dikrotischen Kerbenpunkt in der Blutdruckwellenform der Testperson, der im Voraus berechnet oder gemessen wurde, eingestellt wird; und
Erfassung der zeitlichen Veränderung des Blutdrucks auf Basis der korrigierten Zeitwellenform.

2. Blutdruckmessungsverfahren zum Erfassen einer zeitlichen Änderung eines Blutdrucks, wobei das Blutdruckmessungsverfahren umfasst:

einen Pulswellenerfassungsschritt zum Erfassen einer Zeitwellenform basierend auf einer Volumenpulswelle, wobei die Zeitwellenform eine Wellenform der Volumenpulswelle oder eine Wellenform ist, die durch zeitliches Differenzieren der Volumenpulswelle erhalten wird;
einen DN-Punkt-Erkennungsschritt zum Erkennen eines DN-Punktes, der einem dikrotischen Kerbenpunkt in einer Blutdruckwellenform einer Testperson entspricht, der die zeitliche Änderung des Blutdrucks in der Zeitwellenform angibt; und
einen Wellenformkorrekturschritt zum Korrigieren der Zeitwellenform durch Multiplizieren der Zeitwellenform mit einem Multiplikationskoeffizienten, so dass ein Zeitwellenformwert am DN-Punkt zu einem vorbestimmten Blutdruckwert wird, der im Voraus auf Basis des Blutdruckwertes am dikrotischen Kerbenpunkt in der Blutdruckwellenform der Testperson eingestellt wird, der im Voraus berechnet oder gemessen wurde, wobei die korrigierte Zeitwellenform durch Addieren eines Additionskoeffizienten zu einem korrigierten Zeitwellenformwert zu jedem Zeitpunkt weiter korrigiert wird, so dass ein Verhältnis zwischen einem Minimalwertpunkt und einem Maximalwertpunkt der korrigierten Zeitwellenform zu einem vorbestimmten Wert wird, der im Voraus auf Basis eines im Voraus berechneten oder gemessenen Verhältnisses zwischen einem diastolischen Blutdruck und einem systolischen Blutdruck in der Blutdruck-Wellenform der Testperson eingestellt wird; und
Erfassen der zeitlichen Veränderung des Blutdrucks auf Basis der korrigierten Zeitwellenform.

3. Blutdruckmessungsverfahren nach Anspruch 1 oder 2, wobei die Zeitwellenform eine Wellenform der Volumenpulswelle umfasst.

4. Blutdruckmessungsverfahren nach Anspruch 1 oder 2, wobei die Zeitwellenform eine Wellenform umfasst, die durch Differenzierung der Volumenpulswelle in Bezug auf die Zeit erhalten wird.

5. Blutdruckmessungsverfahren nach einem der Ansprüche 1 bis 4, ferner umfassend:
einen Blutdruckberechnungsschritt zum Berechnen eines Blutdruckwerts auf Basis der korrigierten Zeitwellenform.

6. Blutdruckmessungsverfahren nach Anspruch 5, ferner umfassend:
einen Anzeigeschritt zum Anzeigen mindestens eines der Zeitwellenform, der korrigierten Zeitwellenform und des Blutdruckwerts.

7. Blutdruckmessungsvorrichtung, die zum Erfassen einer zeitlichen Änderung des Blutdrucks konfiguriert ist, wobei die Blutdruckmessungsvorrichtung umfasst:

eine Pulswellenerfassungseinheit, die zum Erfassen einer Zeitwellenform basierend auf einer Volumenpulswelle konfiguriert ist, wobei die Zeitwellenform eine Wellenform ist, die durch Addieren eines Additionskoeffizienten zu einem Volumenpulswellenformwert zu jeder Zeit erhalten wird, wobei die Volumenpulswellenform eine Wellenform der Volumenpulswelle oder eine Wellenform ist, die durch Differenzieren der Volumenpulswelle in Bezug auf die Zeit erhalten wird, so dass ein Verhältnis zwischen einem Minimalwertpunkt und einem Maximalwertpunkt der Volumenpulswellenform zu einem vorbestimmten Wert wird, der im Voraus auf Basis eines im Voraus berechneten oder gemessenen Verhältnisses zwischen einem diastolischen Blutdruck und einem systolischen Blutdruck in einer Blutdruckwellenform einer Testperson festgelegt wird; und

eine Wellenformkorrektureinheit, die zum Erkennen eines DN-Punkts konfiguriert ist, der einem dikrotischen Kerbenpunkt in der Blutdruckwellenform der Testperson entspricht, der die Änderung des Blutdrucks in Bezug auf die Zeit angibt, in der Zeitwellenform und zum Korrigieren der Zeitwellenform durch Multiplizieren der Zeitwellenform mit einem Multiplikationskoeffizienten, so dass ein Zeitwellenformwert am DN-Punkt zu einem vorbestimmten Blutdruckwert wird, der im Voraus auf Basis des Blutdruckwerts am dikrotischen Kerbenpunkt in der Blutdruckwellenform der Testperson, der im Voraus berechnet oder gemessen wurde, eingestellt wird, und Erfassen der zeitlichen Änderung des Blutdrucks auf Basis der korrigierten Zeitwellenform.

8. Blutdruckmessungsvorrichtung, die zum Erfassen einer zeitlichen Änderung des Blutdrucks konfiguriert ist, wobei das Blutdruckmessungsvorrichtung umfasst:

eine Pulswellenerfassungseinheit, die zum Erfassen einer Zeitwellenform basierend auf einer Volumenpulswelle konfiguriert ist, wobei die Zeitwellenform eine Wellenform der Volumenpulswelle oder eine Wellenform ist, die durch Differenzieren der Volumenpulswelle in Bezug auf die Zeit erhalten wird; und

eine Wellenformkorrektureinheit, die zum Erkennen eines DN-Punkts konfiguriert ist, der einem dikrotischen Kerbenpunkt in einer Blutdruckwellenform einer Testperson entspricht, der die zeitlich Änderung des Blutdrucks angibt, in der Zeitwellenform und Korrigieren der Zeitwellenform durch Multiplizieren der Zeitwellenform mit einem Multiplikationskoeffizienten, so dass ein Zeitwellenformwert am DN-Punkt zu einem vorbestimmten Blutdruckwert wird, der im Voraus auf Basis des im Voraus berechneten oder gemessenen Blutdruckwerts am dikrotischen Kerbenpunkt in der Blutdruckwellenform der Testperson eingestellt wird, wobei die korrigierte Zeitwellenform weiter durch Addieren eines Additionskoeffizienten zu einem konjugierten Zeitwellenformwert zu jedem Zeitpunkt korrigiert wird, so dass ein Verhältnis zwischen einem Minimalwertpunkt und einem Maximalwertpunkt der korrigierten Zeitwellenform zu einem vorbestimmten Wert wird, der im Voraus auf Basis eines im Voraus berechneten oder gemessenen Verhältnisses zwischen einem diastolischen Blutdruck und einem systolischen Blutdruck in der Blutdruckwellenform der Testperson, eingestellt wird, und Erfassen der zeitlichen Änderung des Blutdrucks auf Basis der korrigierten Zeitwellenform.

9. Blutdruckmessungsvorrichtung nach Anspruch 7 oder 8, wobei die Zeitwellenform eine Wellenform der Volumenpulswelle umfasst.

10. Blutdruckmessungsvorrichtung nach Anspruch 7 oder 8, wobei die Zeitwellenform die Wellenform umfasst, die durch Differenzieren einer Volumenpulswelle in Bezug auf die Zeit erhalten wird.

11. Blutdruckmessungsvorrichtung nach Anspruch 7 oder 8, ferner umfassend:
eine Blutdruckberechnungseinheit, die zum Berechnen eines Blutdruckwerts auf Basis der korrigierten Zeitwellenform konfiguriert ist.

12. Blutdruckmessungsvorrichtung nach Anspruch 11, ferner umfassend:
eine Anzeigeeinheit, die zum Anzeigen mindestens eines der Zeitwellenform, der korrigierten Zeitwellenform und des Blutdruckwertes konfiguriert ist.

13. Blutdruckmessungsvorrichtung nach einem der Ansprüche 7 bis 12, wobei die Pulswellenerfassungseinheit umfasst:

eine Bestrahlungsvorrichtung, die zum Einstrahlen von Licht in einen lebenden Körper konfiguriert ist; und
einen Photodetektor, der zum Erkennen des durch das Innere des lebenden Körpers durchgelassenen Lichts konfiguriert ist.

14. Blutdruckmessungsprogramm umfassend Anweisungen, die, wenn das Programm von einem Computer ausgeführt wird, bewirken, dass der Computer die Schritte des Verfahrens nach einem der Ansprüche 1 bis 6 ausführt.

**15.** Ein computerlesbares Speichermedium umfassend Anweisungen, die, wenn sie von einem Computer ausgeführt werden, bewirken, dass der Computer das Verfahren nach einem der Ansprüche 1 - 6 ausführt.

**Revendications**

1. Procédé de mesure de pression artérielle pour acquérir un changement d'une pression artérielle par rapport au temps, le procédé de mesure de pression artérielle comprenant :

   une étape d'acquisition d'onde de pulsation consistant à acquérir une forme d'onde temporelle sur la base d'une onde de pulsation volumique, où la forme d'onde temporelle est une forme d'onde obtenue par addition d'un coefficient d'addition à une valeur de forme d'onde de l'onde de pulsation volumique à chaque instant, la forme d'onde de l'onde de pulsation volumique étant une forme d'onde de l'onde de pulsation volumique ou une forme d'onde obtenue par différenciation de l'onde de pulsation volumique par rapport au temps, de sorte qu'un rapport entre un point de valeur minimale et un point de valeur maximale de la forme d'onde de l'onde de pulsation volumique devienne une valeur prédéterminée qui est réglée à l'avance sur la base d'un rapport entre une pression artérielle diastolique et une pression artérielle systolique dans une forme d'onde de la pression artérielle d'un sujet de test calculé ou mesuré à l'avance ;
   une étape de détection de point DN consistant à détecter un point DN, qui correspond à un point d'encoche dicrote dans la forme d'onde de la pression artérielle du sujet de test indiquant le changement de la pression artérielle par rapport au temps, dans la forme d'onde temporelle ; et
   une étape de correction de forme d'onde consistant à corriger la forme d'onde temporelle par multiplication de la forme d'onde temporelle par un coefficient de multiplication de sorte qu'une valeur de forme d'onde temporelle au point DN devienne une valeur de pression artérielle prédéterminée qui est réglée à l'avance sur la base de la valeur de pression artérielle au point d'encoche dicrote dans la forme d'onde de la pression artérielle du sujet de test calculée ou mesurée à l'avance ; et
   à acquérir le changement de la pression artérielle par rapport au temps sur la base de la forme d'onde temporelle corrigée.

2. Procédé de mesure de pression artérielle pour acquérir un changement d'une pression artérielle par rapport au temps, le procédé de mesure de pression artérielle comprenant :

   une étape d'acquisition d'onde de pulsation consistant à acquérir une forme d'onde temporelle sur la base d'une onde de pulsation volumique, où la forme d'onde temporelle est une forme d'onde de l'onde de pulsation volumique ou une forme d'onde obtenue par différenciation de l'onde de pulsation volumique par rapport au temps ;
   une étape de détection de point DN consistant à détecter un point DN, qui correspond à un point d'encoche dicrote dans une forme d'onde de la pression artérielle d'un sujet de test indiquant le changement de la pression artérielle par rapport au temps, dans la forme d'onde temporelle ; et
   une étape de correction de forme d'onde consistant à corriger la forme d'onde temporelle par multiplication de la forme d'onde temporelle par un coefficient de multiplication de sorte qu'une valeur de forme d'onde temporelle au point DN devienne une valeur de pression artérielle prédéterminée qui est réglée à l'avance sur la base de la valeur de pression artérielle au point d'encoche dicrote dans la forme d'onde de la pression artérielle du sujet de test calculée ou mesurée à l'avance, où la forme d'onde temporelle corrigée est en outre corrigée par addition d'un coefficient d'addition à une valeur de forme d'onde temporelle corrigée à chaque instant, de sorte qu'un rapport entre un point de valeur minimale et un point de valeur maximale de la forme d'onde temporelle corrigée devienne une valeur prédéterminée qui est réglée à l'avance sur la base d'un rapport entre une pression artérielle diastolique et une pression artérielle systolique dans la forme d'onde de la pression artérielle du sujet de test calculé ou mesuré à l'avance ; et
   à acquérir le changement de la pression artérielle par rapport au temps sur la base de la forme d'onde temporelle corrigée.

3. Procédé de mesure de pression artérielle selon la revendication 1 ou 2, dans lequel la forme d'onde temporelle comprend une forme d'onde de l'onde de pulsation volumique.

4. Procédé de mesure de pression artérielle selon la revendication 1 ou 2, dans lequel la forme d'onde temporelle comprend une forme d'onde obtenue par différenciation de l'onde de pulsation volumique par rapport au temps.

**5.** Procédé de mesure de pression artérielle selon l'une quelconque des revendications 1 à 4, comprenant en outre :
une étape de calcul de pression artérielle consistant à calculer une valeur de pression artérielle sur la base de la forme d'onde temporelle corrigée.

**6.** Procédé de mesure de pression artérielle selon la revendication 5, comprenant en outre :
une étape d'affichage consistant à afficher au moins l'une de la forme d'onde temporelle, de la forme d'onde temporelle corrigée et de la valeur de pression artérielle.

**7.** Dispositif de mesure de pression artérielle configuré pour acquérir un changement d'une pression artérielle par rapport au temps, le dispositif de mesure de pression artérielle comprenant :

une unité d'acquisition d'onde de pulsation configurée pour acquérir une forme d'onde temporelle sur la base d'une onde de pulsation volumique, où la forme d'onde temporelle est une forme d'onde obtenue par addition d'un coefficient d'addition à une valeur de forme d'onde de l'onde de pulsation volumique à chaque instant, la forme d'onde de l'onde de pulsation volumique étant une forme d'onde de l'onde de pulsation volumique ou une forme d'onde obtenue par différenciation de l'onde de pulsation volumique par rapport au temps, de sorte qu'un rapport entre un point de valeur minimale et un point de valeur maximale de la forme d'onde de l'onde de pulsation volumique devienne une valeur prédéterminée qui est réglée à l'avance sur la base d'un rapport entre une pression artérielle diastolique et une pression artérielle systolique dans une forme d'onde de la pression artérielle d'un sujet de test calculé ou mesuré à l'avance ; et
une unité de correction de forme d'onde configurée pour détecter un point DN, qui correspond à un point d'encoche dicrote dans la forme d'onde de la pression artérielle du sujet de test indiquant le changement de la pression artérielle par rapport au temps, dans la forme d'onde temporelle et pour corriger la forme d'onde temporelle par multiplication de la forme d'onde temporelle par un coefficient de multiplication de sorte qu'une valeur de forme d'onde temporelle au point DN devienne une valeur de pression artérielle prédéterminée qui est réglée à l'avance sur la base de la valeur de pression artérielle au point d'encoche dicrote dans la forme d'onde de la pression artérielle du sujet de test calculée ou mesurée à l'avance et pour acquérir le changement de la pression artérielle par rapport au temps sur la base de la forme d'onde temporelle corrigée.

**8.** Dispositif de mesure de pression artérielle configuré pour acquérir un changement d'une pression artérielle par rapport au temps, le dispositif de mesure de pression artérielle comprenant :

une unité d'acquisition d'onde de pulsation configurée pour acquérir une forme d'onde temporelle sur la base d'une onde de pulsation volumique, où la forme d'onde temporelle est une forme d'onde de l'onde de pulsation volumique ou une forme d'onde obtenue par différenciation de l'onde de pulsation volumique par rapport au temps ; et
une unité de correction de forme d'onde configurée pour détecter un point DN, qui correspond à un point d'encoche dicrote dans une forme d'onde de la pression artérielle d'un sujet de test indiquant le changement de la pression artérielle par rapport au temps, dans la forme d'onde temporelle et pour corriger la forme d'onde temporelle par multiplication de la forme d'onde temporelle par un coefficient de multiplication de sorte qu'une valeur de forme d'onde temporelle au point DN devienne une valeur de pression artérielle prédéterminée qui est réglée à l'avance sur la base de la valeur de pression artérielle au point d'encoche dicrote dans la forme d'onde de la pression artérielle du sujet de test calculée ou mesurée à l'avance, où la forme d'onde temporelle corrigée est en outre corrigée par addition d'un coefficient d'addition à une valeur de forme d'onde temporelle corrigée à chaque instant, de sorte qu'un rapport entre un point de valeur minimale et un point de valeur maximale de la forme d'onde temporelle corrigée devienne une valeur prédéterminée qui est réglée à l'avance sur la base d'un rapport entre une pression artérielle diastolique et une pression artérielle systolique dans la forme d'onde de la pression artérielle du sujet de test calculé ou mesuré à l'avance et pour acquérir le changement de la pression artérielle par rapport au temps, sur la base de la forme d'onde temporelle corrigée.

**9.** Dispositif de mesure de pression artérielle selon la revendication 7 ou 8, dans lequel la forme d'onde temporelle comprend une forme d'onde de l'onde de pulsation volumique.

**10.** Dispositif de mesure de pression artérielle selon la revendication 7 ou 8, dans lequel la forme d'onde temporelle comprend la forme d'onde obtenue par différenciation d'une onde de pulsation volumique par rapport au temps.

**11.** Dispositif de mesure de pression artérielle selon la revendication 7 ou 8, comprenant en outre :
une unité de calcul de pression artérielle configurée pour calculer une valeur de pression artérielle sur la base de la forme d'onde temporelle corrigée.

12. Dispositif de mesure de pression artérielle selon la revendication 11, comprenant en outre :
une unité d'affichage configurée pour afficher au moins l'une de la forme d'onde temporelle, de la forme d'onde temporelle corrigée, et de la valeur de pression artérielle.

13. Dispositif de mesure de pression artérielle selon l'une quelconque des revendications 7 à 12, dans lequel l'unité d'acquisition d'onde de pulsation comprend :

un dispositif d'exposition à un rayonnement configuré pour rayonner de la lumière à l'intérieur d'un corps vivant ; et
un photodétecteur configuré pour détecter la lumière transmise à travers l'intérieur du corps vivant.

14. Programme de mesure de pression artérielle comprenant des instructions qui, lorsque le programme est exécuté par un ordinateur, amènent l'ordinateur à exécuter les étapes du procédé de l'une des revendications 1 à 6.

15. Support de stockage lisible par ordinateur comprenant des instructions qui, lorsqu'elles sont exécutées par un ordinateur, amènent l'ordinateur à mettre en œuvre le procédé de l'une des revendications 1 à 6.

## Fig.1

*Fig.2*

1

20

PULSE WAVE
MEASUREMENT DEVICE — 10

PULSE WAVE
ACQUISITION UNIT — 21

WAVEFORM
CORRECTION UNIT — 22

BLOOD PRESSURE
CALCULATION UNIT — 23

DISPLAY UNIT — 24

**Fig.3**

(a)

(b)

# *Fig.4*

(a)

(b)

# *Fig.5*

BLOOD FLOW

V

A

**Fig.6**

(a)

(b)

ABOUT 1:1.5

EP 3 210 529 B1

# Fig.7

EP 3 210 529 B1

(a)

ABOUT 1:1.5

(b)

ABOUT 90mmHg

× MULTIPLICATION COEFFICIENT

# Fig.8

# Fig.9

```
                    ┌─────────────┐
                    │    START    │
                    └─────────────┘
                           │
        ┌──────────────────────────────────────┐
        │   ACQUIRE PULSE WAVE WAVEFORM         │ ～S1
        └──────────────────────────────────────┘
                           │
        ┌──────────────────────────────────────┐
        │   CORRECT PULSE WAVE WAVEFORM         │ ～S2
        └──────────────────────────────────────┘
                           │
        ┌──────────────────────────────────────┐
        │  CALCULATE BLOOD PRESSURE VALUE       │
        │    ON THE BASIS OF WAVEFORM           │ ～S3
        │       CORRESPONDING TO                │
        │   BLOOD PRESSURE WAVEFORM             │
        └──────────────────────────────────────┘
                           │
        ┌──────────────────────────────────────┐
        │  DISPLAY PULSE WAVE WAVEFORM,         │
        │  WAVEFORM CORRESPONDING TO            │ ～S4
        │  BLOOD PRESSURE WAVEFORM,             │
        │  OR BLOOD PRESSURE VALUE              │
        └──────────────────────────────────────┘
                           │
                    ┌─────────────┐
                    │     END     │
                    └─────────────┘
```

# Fig.10

$$\text{CORRECTION PROCESS START}$$

DETECT MINIMUM POINT $Q_{min}$
AND MAXIMUM POINT $Q_{max}$
IN PULSE WAVE WAVEFORM — S21

CORRECT PULSE WAVE WAVEFORM
ON THE BASIS OF RATIO
BETWEEN MINIMUM POINT $Q_{min}$
AND MAXIMUM POINT $Q_{max}$ — S22

DETECT DN POINT $Q_{DN}$
IN PULSE WAVE WAVEFORM — S23

CORRECT PULSE WAVE WAVEFORM
ON THE BASIS OF PULSE WAVE
WAVEFORM VALUE OF DN POINT $Q_{DN}$ — S24

$$\text{CORRECTION PROCESS END}$$

# *Fig.11*

EP 3 210 529 B1

# Fig.12

# Fig.13

## Fig.14

# Fig.15

Fig.16

## Fig.17

# *Fig.18*

**REFERENCES CITED IN THE DESCRIPTION**

**Patent documents cited in the description**

- JP 2006006897 A
- JP 11155826 A
- JP 2001187032 A
- US 20120277602 A